# FASCICULE DE BREVET EUROPEEN

(11) **EP 1 866 651 B1**
(45) Date de publication et mention de la délivrance du brevet: **12.05.2010**
(21) Numéro de dépôt: 06726120.6
(22) Date de dépôt: 22.03.2006
(51) Int. Cl.: G01N 33/72, G01N 33/50

(54) **PROCÉDÉ POUR L'ANALYSE D'UN ÉCHANTILLON DE SANG**
VERFAHREN ZUR ANALYSE EINER BLUTPROBE
METHOD FOR ANALYZING A BLOOD SAMPLE

(30) Priorité: 31.03.2005 FR 0503118
(43) Date de publication de la demande: 19.12.2007
(73) Titulaire: C2 DIAGNOSTICS, 34099 Montpellier (FR)
(72) Inventeur: CHAMPSEIX, Henri, F-34980 Saint Gely du Fesc (FR); GIRAUD, Jérôme, 34090 Montpellier (FR)
(74) Mandataire: Pontet, Bernard
(86) Numéro de dépôt international: PCT/FR2006/000622
(87) Numéro de publication internationale: WO 2006/103334

(56) Documents cités:
- EP-A- 0 430 750
- EP-A- 0 978 724
- US-A- 4 529 705
- US-A- 5 250 437
- US-A- 5 510 267
- US-A- 5 786 224
- US-A- 5 958 781
- US-E1- R E38 131

## Description

La présente invention concerne un procédé pour l'analyse d'un échantillon de sang. L'invention vise plus particulièrement le domaine des analyses automatiques d'échantillons de sang.

On recherche par l'analyse d'un échantillon de sang à déterminer généralement :
- le nombre total de globules blancs ;
- plus spécifiquement, le nombre de globules blancs par sous populations (basophiles, éosinophiles, neutrophiles, monocytes et lymphocytes) ;
- le nombre de globules rouges et de plaquettes ; et
- le taux d'hémoglobine.

Plusieurs techniques d'analyse sont connues, notamment :
- le dosage de l'hémoglobine est effectué après la lyse des globules rouges, c'est-à-dire la destruction de la membrane des cellules de globules rouges, et par mesure par spectrophotométrie de l'hémoglobine libérée dans le milieu ; le dosage de l'hémoglobine nécessite en outre la stabilisation de l'hémoglobine sous une forme complexée (oxyhémoglobine ou cyanméthémoglobine) de manière à mesurer l'absorbance d'un seul composé à la longueur d'onde appropriée.
- le comptage total des globules blancs est effectué sur l'échantillon de sang par résistivité en réalisant la lyse spécifique des globules rouges et la protection des globules blancs.
- la différenciation des globules blancs et leur comptage par sous population sont effectués :
   - soit par mesure volumétrique en résistivité après lyse spécifique des globules rouges, protection des globules blancs et réglage du pH ; cela ne permettant cependant pas de différentier toutes les sous populations en une seule analyse ;
   - soit par voie optique, notamment par cytométrie de flux ; après lyse spécifique des globules rouges et protection des globules blancs, en mesurant différents paramètres (notamment diffraction, fluorescence, absorbance) sur un flux de globules blancs dans l'axe au petits, moyens et grands angles et éventuellement après ajout d'un agent de marquage (par exemple du noir de chlorazol, ou un colorant marquant l'ADN ou l'ARN, ou un colorant fluorescent) et par mesure à différentes longueurs d'onde ; cette technique permettant de différentier les sous-populations de globules blancs.
- le comptage des globules rouges et des plaquettes se fait sur un échantillon dilué sans ajout de réactif particulier par mesure de résistivité.

Il existe de nombreux automates d'hématologie qui mettent en oeuvre ces techniques afin d'obtenir une analyse la plus complète possible d'un échantillon de sang.

Dans ces automates, coexistent traditionnellement deux circuits d'analyse différents :
- un premier circuit conçu pour la mesure de l'hémoglobine et/ou le comptage total des globules blancs ; et
- un deuxième circuit conçu pour réaliser sur l'échantillon de sang la différenciation et/ou un comptage leucocytaire par cytométrie de flux.

Chaque circuit est caractérisé par un taux de dilution de l'échantillon de sang adapté aux moyens de mesure utilisés, l'ajout d'un ou plusieurs réactifs et des moyens de mise en oeuvre et de mesure appropriés.

Ainsi, pour la mesure de l'hémoglobine et le comptage des globules blancs, le circuit comporte typiquement une cuve dite de comptage dans laquelle on dilue l'échantillon de sang, on y ajoute un réactif comprenant notamment le composé de lyse des globules rouges, le composé de stabilisation du complexe formé de l'hémoglobine et le composé leucoprotecteur, et on mesure directement dans cette cuve l'hémoglobine par spectrophotométrie et le nombre de globules blancs par résistivité. Le taux de dilution est choisi de sorte que la solution d'analyse soit parfaitement homogène et de sorte que l'appareil de détection ne soit pas saturé. Ce taux de dilution est compris entre 1/100e et 1/500^{e}, en général entre 1/160^{e} et 1/180^{e}.

Pour la différenciation leucocytaire par cytométrie de flux, le circuit met en oeuvre une cuve de dilution de l'échantillon de sang dans laquelle on ajoute un ou plusieurs réactif contenant un agent de lyse des globules rouges, éventuellement un agent de différenciation (par exemple un colorant fluorescent de l'ADN ou l'ARN des globules blancs), puis on prélève une fraction de cette solution pour l'injecter dans une cuve optique de circulation d'un cytomètre de flux. Ici, le taux de dilution pratiqué est inférieur à 1/100^{e}, permettant d'obtenir avec les cytomètres connus sur le marché actuellement (de type hydrofocus) un temps d'analyse optimum.

Ainsi, traditionnellement, au moins deux réactifs différents doivent être utilisés pour les deux circuits d'analyse et deux dilutions différentes de l'échantillon de sang sont pratiquées dans ces deux circuits d'analyse.

Les objectifs principalement visés par les fabricants sont de simplifier les automates existants en réduisant le nombre de composants et de réactifs, cela permettant de réduire les coûts de fabrication, de maintenance et la taille des automates, sans pour autant réduire les temps d'une analyse complète d'un échantillon de sang.

La présente invention tend notamment à atteindre ces objectifs.

Le document WO 2004/003517 propose dans ce sens un procédé et un appareillage où les deux circuits d'analyse ont des moyens communs. Le principe est de réaliser une première dilution de l'échantillon de sang dans un bac unique de dilution et de transférer successivement des fractions de volumes choisis de cette dilution vers un ensemble de mesure ou de comptage, pour mesurer ou compter à chaque fois des éléments différents contenus dans l'échantillon de sang. Pour réaliser une analyse complète, à savoir un comptage des globules rouges et des plaquettes, un comptage des globules blancs, une mesure de l'hémoglobine et une différenciation leucocytaire, le document décrit la solution suivante : utiliser un premier transfert pour compter les globules rouges et les plaquettes, ajouter un agent de lyse dans le bac de dilution, puis réaliser un deuxième transfert pour réaliser le comptage des globules blancs, réaliser un troisième transfert de solution de dilution lysée pour mesurer le taux d'hémoglobine, ajouter un réactif de différenciation leucocytaire, et réaliser un quatrième transfert pour réaliser la différenciation leucocytaire dans l'ensemble de mesure.

Ce principe permet certes de mettre en oeuvre une seule cuve dite de dilution, mais il ne permet pas un gain du temps d'analyse puisque les mesures ou comptage sont réalisés successivement après chaque transfert d'une fraction de la dilution. En outre, il nécessite un contrôle parfait des volumes de réactifs successifs et de diluants distribués ainsi que des volumes de dilution transférés vers l'ensemble de mesure. Il nécessite en outre encore la mise en oeuvre de plusieurs seringues et réactifs de lyse.

Le document US 5 958 781 A décrit un procédé d'analyse d'un échantillon de sang utilisant un mono-réactif de lyse fonctionnant en tandem avec un diluant spécifique permettant la mesure de l'hémoglobine et l'énumération d'au moins trois sous-populations leucocytaires, possiblement par voie optique, bien qu'aucune donnée du texte n'étaye l'utilisation de moyens optiques de détection et les résultats associés. Ce procédé repose sur l'emploi d'un diluant déterminé dont la composition est indiquée.

Le document US 5 786 224 A décrit un procédé d'analyse d'un échantillon de sang utilisant une composition de bi-réactifs comme agent de lyse, permettant la différenciation d'au moins trois sous populations de leucocytes. Ce procédé ne fait pas intervenir de dilution préalable avec un diluant.

Le document US 4529 705 A décrit un procédé d'analyse d'un échantillon de sang utilisant un unique réactif permettant à la fois d'effectuer la dilution, et la lyse des globules rouges, et contenant un agent complexant l'hémoglobine, spécifiquement l'ion cyanure (ou son complexe ferricyanure) dont la toxicité n'est plus à démontrer. En outre, l'énumération globulaire proposée par ce procédé permet de quantifier que le nombre total de globules blancs mais pas la différentiation des sous-populations, et ce par voie électronique résistive uniquement (aucunement optique).

Le document US RE38 131 E décrit également un procédé d'analyse d'un échantillon de sang utilisant un mono-réactif permettant la détermination de deux ou trois sous-populations leucocytaires, mais uniquement par voie résistive.

La présente invention a en outre pour objectif de pallier de tels inconvénients.

La présente invention vise un procédé d'analyse automatique d'un échantillon de sang, dans lequel :
* on forme, dans un bac unique dit de dilution et d'analyse, une solution d'analyse contenant :
   - ledit échantillon de sang,
   - un diluant,
   - un mono réactif comprenant au moins un composé pour lyser les globules rouges, au moins un composé pour stabiliser l'hémoglobine sous forme d'oxyhémoglobine et au moins un composé pour protéger les globules blancs, permettant la discrimination d'au moins cinq principales sous-populations leucocytaires, ledit composé pour protéger les globules blancs étant choisi parmi les bétaïnes et sulfo-bétaïnes d'ammoniums quaternaires, les oxydes d'aminés tertiaires, les composés de type glycosidiques et les composés de type glucidiques,
* on mesure sur cette solution d'analyse dans ledit bac après la lyse des globules rouges le taux d'hémoglobine par spectrophotométrie ; et
* on prélève une quantité appropriée de cette solution d'analyse dans ledit bac sur laquelle on réalise une différenciation leucocytaire d'au moins 5 sous-populations par un moyen optique.

Le procédé selon l'invention est **caractérisé en ce que** la solution d'analyse contient en outre au moins un composé pour protéger les globules blancs, permettant la discrimination des cinq principales sous-populations leucocytaires.

Le comptage des globules blancs peut être réalisé conjointement dans le bac d'analyse et/ou par le moyen optique.

Le comptage des globules rouges et éventuellement des plaquettes peut être réalisé par exemple dans une étape préalable du procédé sur un prélèvement effectué dans le bac unique de dilution et d'analyse.

Ainsi, la présente invention est basée sur le concept d'une solution unique d'analyse utilisée telle quelle pour les deux types d'analyses qui étaient traditionnellement menées dans deux circuits séparés, à savoir d'une part la mesure de l'hémoglobine et éventuellement le comptage des globules blancs et, d'autre part, la différenciation leucocytaire par un moyen optique, ladite solution d'analyse réunissant les composés « réactifs » capables de mener à bien au moins ces analyses, de par leur nature et leur quantité. Les composés réactifs introduits sont choisis pour être chimiquement compatibles entre eux et en quantités adaptées aux analyses visées. Ils peuvent être choisis parmi les composés typiquement utilisés dans l'art antérieur. On peut aussi utiliser une formulation commerciale utilisée classiquement pour réaliser une différentiation leucocytaire, c'est-à-dire contenant le composé pour lyser les globules rouges et le composé leucoprotecteur, et lui ajouter le troisième composé réactif destiné à stabiliser l'hémoglobine sous la forme d'un complexe chromogène.

Grâce à cette solution d'analyse unique, la présente invention présente notamment les avantages suivants :
- l'automate peut comporter un bac unique de préparation de la solution d'analyse,
- la mesure de l'hémoglobine peut être réalisée directement sur ce bac, ainsi que le comptage global des globules blancs par mesure de résistivité de la solution d'analyse ;
- on peut mettre en oeuvre un monoréactif réunissant tous les composés « réactifs » nécessaires pour la mesure de l'hémoglobine et pour la différenciation leucocytaire par un moyen optique ; ceci permet notamment de simplifier les circuits hydrauliques comme on le verra plus loin ;
- on peut réaliser une mono-dilution de l'échantillon de sang directement dans le bac unique de dilution et d'analyse, avec un taux de dilution déterminé en fonction des moyens de mesure et de détection utilisés. Le mono-réactif peut servir de diluant pour réaliser cette monodilution. De préférence, on fixera un taux de dilution compris entre 1/100e et 1/500e, correspondant au taux de dilution nécessaire à une mesure du taux d'hémoglobine, de préférence encore un taux environ de 1/175^{e} (1/173^{e} dans l'exemple de réalisation donné plus loin).

Avec la possibilité de mettre en oeuvre une mono-dilution et un mono-réactif, on peut donc, grâce à ce premier aspect de l'invention, simplifier grandement les appareillages d'analyse tout en offrant toujours la possibilité de réaliser une analyse complète de l'échantillon de sang.

Des moyens de mesure optique permettant d'obtenir une analyse des globules blancs (comptage et différenciation par sous populations) à un taux de dilution supérieur à 1/100^{e} sont proposés également selon l'invention et sont définis et décrits plus loin.

Le monoréactif utilisé dans le procédé selon l'invention permet la mesure par spectrophotométrie de la concentration en hémoglobine d'un échantillon de sang et une différentiation leucocytaire par un moyen optique. Il permet en outre le comptage résistif et/ou optique des globules blancs. De manière préférentielle, il est choisi pour permettre la différenciation d'au moins 5 sous-populations. De manière préférentielle, il est choisi de façon à ne pas contenir de cyanures.

Selon l'invention, le composé pour lyser les globules rouges est de préférence constitué d'au moins un surfactant cationique.

Dans la présente invention on choisit de former un complexe oxyhémoglobine (car non toxique par comparaison à un complexe cyanméthémoglobine mettant en oeuvre des ions cyanures). Le surfactant cationique est donc choisi de manière en outre à oxyder l'hémoglobine libérée pour ne former qu'un complexe oxyhémoglobine. La quantité de surfactant cationique est donc choisie de manière à hémolyser efficacement les globules rouges et oxyder l'hémoglobine libérée. Il est de préférence choisi parmi :
- les sels d'ammoniums quaternaires, de préférence les sels d'alkyltriméthylammoniums et plus particulièrement encore les bromures et chlorures de cétyl-, dodécyl-, tétradécyl- et hexadécyltriméthylammonium ;
- les sels de pyridiniums ;
- les amines à longues chaînes éthoxylées ; et
- les alkylsulfates (SDS).

Le composé leucoprotecteur selon l'invention est un composé retardant ou prévenant la destruction des globules blancs. De préférence, il s'agit d'un surfactant non ionique ou amphotère choisi de préférence parmi :
- les alcools éthoxylés, notamment le 2-phénoxyéthanol, les polyoxyéthylènealkylphényléther, comme les produits commercialisés IPEGAL990^{®}, TERGITOL NP9^{®}, TRITON^{®} X100 ou X114, plurafac^{®} A38 ou Brij35^{®}) ;
- les bétaïnes et sulfo-bétaïnes d'ammoniums quaternaires notamment la lauramidopropyl bétaïne (LAB), et le dodécyldiméthyl-3-ammonio-1-propanesulfonate (DDAPS) ou le tétradécyldiméthyl-3-ammonio-1-propanesulfonate (TDAPS) ;
- les oxydes d'amines tertiaires, comme le N,N-diméthyllaurylamine-N-oxyde (LDAO) ou le sulfonate de 3-[(3cholamidopropyl)-diméthylamino-]-1-propane (CHAPS ou CHAPSO) ;
- les composés de type glycosidiques et plus particulièrement une saponine triterpénique ;
- les composés de type glucidiques (mannitol, D-glucose, tréhalose, sulfate de dextran).

Le composé stabilisant l'hémoglobine sous la forme d'un complexe chromogène d'oxyhémoglobine est de préférence choisi parmi :
- les chélates mono ou polydendates présentant des atomes ligands (doublets non liants : O, N, S, et groupements carboxy COO-...) notamment :
   - les sels de l'acide éthylènediaminetétraacétique (EDTA) ou de l'acide éthylèneglycol-bis-(3-aminoéthyléther)N-N'-tétraacétique (EGTA) et notamment leurs sels disodiques ou dipotassiques ;
   - l'oxalate de potassium K₂Oₓ Oₓ=C₂O₄²⁻ ; et
   - les acides organiques.
- les composés aromatiques (chélates mono ou polydendates) comportant des atomes ligands (présentant des doublets non liants : O, N, S...), notamment :
   - le Tiron® ;
   - la pyridine ou bipyridine et leurs dérivés ;
   - les composés phénoliques (mono ou bis et leurs dérivés) ;
   - le pyrazole et/ou les pyrazolones et leurs dérivés ;
   - l'imidazole et ses dérivés ;
   - l'acide sulfosalicylique ; et
- les saponines, les oxydes d'amines tertiaires, les bétaïnes et sulfo-bétaïnes d'ammoniums quaternaires (comme le DDAPS, TDAPS, LAB).

En outre des trois composés définis selon l'invention, on peut ajouter au(x) (mono)réactif(s) :
- au moins un colorant (ou mélange) marquant spécifiquement certains leucocytes et plus particulièrement les éosinophiles (ou basophiles), pour permettre de discriminer au moins les 5 principales sous-populations leucocytaires, choisi parmi :
   - les cyanines ;
   - l'Oxazine 750 ;
   - les réactifs de Wright et Romanowsky ;
   - le DAPI ;
   - le Noir Chlorazole E ;
   - le bleu de Toluidine ;
   - l'Astra Blue ;
   - et le thiazole orange G. ou blue ;
   - d'autres réactifs fluorescents.
- au moins un agent fixateur permettant de rigidifier la membrane des globules blancs qui sera préférentiellement un aldéhyde et plus particulièrement le glutaraldéhyde ou formaldéhyde ;
- au moins un agent mouillant pour optimiser la fluidique et éviter la formation de bulles agissant en outre comme agent de solubilisation des débris, choisi parmi :
   - les alcools (méthanol éthanol ou propan-2-ol) ;
   - les glycols (éthylène ou propylène glycol) ;
   - les alcools éthoxylés (particulièrement le Triton X100® ou le Brij35®) ;
   - les composés glycosidiques TWEEN80® ou TWEEN20® ;
   la concentration de l'agent de fixation et de solubilisation étant strictement limitée, car un excès peut empêcher la lyse des globules rouges et modifier les propriétés optiques des globules blancs ; et
- un système tampon pour fixer le pH entre 5,0 et 10,0 et de préférence entre 6,0 et 8,0 et de façon optimale proche de la neutralité (7,0±0,4). Le choix d'un tel pH vise à respecter les conditions natives des cellules. En outre ce pH permet une meilleure dissolution des composants mis en oeuvre selon l'invention. Ledit tampon est constitué d'un couple de sels (inorganiques ou organiques) amené au pH précité par de l'acide chlorhydrique ou de la soude (4-6N), choisi parmi :
   - le dihydrogénophosphate/hydrogénophosphate H₂PO₄⁻/HPO₄²- de sodium ou potassium ;
   - l'hydrogénocarbonate/carbonate de sodium NaHCO₃ /Na₂CO₃
   - un tampon acide citrique/citrate de sodium (III)
   - le TRIS-HCl
   - la triéthanolamine (TEA)
   - l'imidazole
- un acide choisi parmi :
   - les acides organiques : phtalique, sulfosalicylique ou formique qui contribuent également à la formation et stabilisation du complexe chromogène de l'hémoglobine) ; et
   - les acides minéraux : HCl, H₃PO₄...
- un sel de fond assurant une conductivité de l'ordre de 10 à 50ms/cm nécessaire pour la mesure de résistivité et une osmolarité de l'ordre de 120 à 500mOsm et préférentiellement proche de l'isotonicité (290±5mOsm), choisi parmi :
   - le chlorure de sodium NaCl ;
   - le chlorure de potassium KCl ;
   - le chlorure de magnésium MgCl₂ ;
   - le chlorure de calcium CaCl₂ ;
   - le sulfate de sodium anhydre Na₂SO₄ ; ce sel de fond pouvant être compris dans le système tampon ;
- au moins un conservateur, ayant des propriétés antioxydantes, et/ou antibiotiques choisi parmi :
   - le 2-phénoxyéthanol ;
   - les parabens ;
   - le BHT ;
   - les isothiazolones (Proclin® 150 ou 300) ;
   - les dérivés de l'imidazole ou de l'urée ;
   - les antibiotiques ;
- un composé de pénétration cellulaire (ionophore) antibiotique naturel qui facilite en outre la pénétration du ou des colorants choisi parmi :
   - l'ionophore I à NH₄⁺ (Nonatine) ;
   - l'ionophore III à Ca²⁺ (Calcimycine) ;
   - l'ionophore à Cl⁻ ;
   - L'ionophore I à K⁺ (Valinomycine).

Les composants selon l'invention sont récapitulés dans le tableau donné ci-après ainsi que des fourchettes de concentrations appropriées.

| Composant | Quantité |
|---|---|
| Surfactant cationique (agent de lyse) | 0,1-50g/L |
| Surfactant leucoprotecteur | 0,1-20g/L |
| Chélate du complexe Hémoglobine | 0,0001-10g/L |
| Colorant | 0,01-1g/L |
| Agent fixateur | 0,01-2%w/v |
| Agent mouillant | 0-50%v/v |
| Tampon | 0-6g/L |
| Sel de fond | 1-50g/L |
| Acide | Quantité appropriée pour fixer le pH |
| Conservateur | Quantité appropriée 0,1-3g/L |
| Ionophore | Quantité efficace 0-200mg/L |
| Eau distillée q.s.p. | qsp 1L |

La présente divulgation propose également un appareil pour la mise en oeuvre du procédé selon l'invention qui est caractérisé par :
- un bac d'analyse adapté à recevoir ladite solution d'analyse ;
- un moyen de mesure par spectrophotométrie dans ledit bac du taux d'hémoglobine présent dans ladite solution d'analyse ;
- un moyen de prélèvement de ladite solution d'analyse ;
- un moyen de mesure optique sur ledit prélèvement afin de réaliser une analyse leucocytaire.

La présente divulgation vise un dispositif optique pour un automate d'analyse automatique d'un échantillon de sang, particulièrement avantageux en outre pour la mise en oeuvre du procédé selon le premier objet de l'invention.

Comme mentionné plus haut, certaines sous-populations de leucocytes ne peuvent être différenciées que par des mesures optiques, par exemple une mesure de diffraction par la cellule à un ou plusieurs angles, ou, une mesure de l'absorbance de la cellule. Les systèmes optiques de caractérisation d'une cellule sanguine ont une base commune dans laquelle on retrouve une source lumineuse émettant un faisceau lumineux, une cuve optique dans laquelle les cellules sanguines traversent le faisceau lumineux, un système de réglage du faisceau lumineux sur le flux de cellules et des moyens de mesure de la lumière issue de la cuve optique après interception par les cellules. Notamment dans le cas de la caractérisation leucocytaire, les globules blancs se déplacent en un flux dans la cuve. Ils y sont éclairés par un faisceau lumineux focalisé sur le flux, que l'on appelle flux échantillon.

De tels dispositifs sont coûteux ; en particulier, les lasers utilisés en tant que sources lumineuses, qui sont en outre encombrants et requièrent généralement un système de dissipation thermique ; les diodes laser, comme les lasers nécessitent des systèmes d'alignement coûteux. Les faisceaux lumineux émis par ces sources ont une répartition transverse de la lumière de forme sensiblement gaussienne. Ainsi, l'intensité n'est sensiblement constante et maximale que dans une partie étroite et centrale du rayon. Les systèmes d'alignement permettent d'aligner cette partie centrale avec le flux d'échantillon. En outre, la largeur du flux d'échantillon ne doit pas excéder celle de cette partie centrale, et la précision du système d'alignement doit être d'autant plus grande que ces deux largeurs sont proches. Il en résulte la nécessité de réduite au maximum la largeur du flux d'échantillon.

Le flux échantillon contenant les cellules sanguines à compter et/ou à différencier doit être d'autant plus étroit que la lumière est focalisée. Ainsi, on utilise un flux dont la largeur de la section est inférieure à 50µm, qui doit traverser le faisceau lumineux lui-même focalisé en un pinceau de section plus grande que celle du flux d'échantillon. Cela nécessite un système d'injection particulièrement précis, donc coûteux, du flux dans la cuve optique. Dans l'art antérieur, on obtient un tel résultat en utilisant un système de type hydrofocus (contraction de l'expression anglaise « hydrodynamic focusing »). On gaine le flux échantillon par un flux de manchonnage. Un injecteur pour le flux échantillon est immergé au coeur du flux de manchonnage. Le flux d'échantillon ainsi créé est étiré, ou focalisé, durant son trajet depuis l'injecteur jusqu'à la zone éclairée par le faisceau lumineux, de sorte qu'il a, à cet endroit, une largeur souhaitée d'environ 5 à 50µm de diamètre. Un simple ou un double manchonnage est parfois nécessaire pour atteindre cet objectif.

En outre, comme on l'a dit précédemment, compte tenu du niveau de précision requis, un système de réglage est indispensable pour faire coïncider le flux de cellules avec le faisceau lumineux. Deux approches sont possibles : on peut déplacer le flux de cellules ou le faisceau lumineux. Si l'on choisit de déplacer le flux de cellules sanguines, il faut déplacer l'ensemble de la cuve optique. Lorsque cette option est retenue, la cuve est montée sur une table à translation qui assure un déplacement fin et uniforme selon deux axes grâce à ses roulements à billes. Un tel ensemble mécanique de précision est assez onéreux. On peut également déplacer le faisceau lumineux pour le faire coïncider avec le flux de cellules sanguines. Ceci est généralement réalisé à l'aide de plusieurs prismes orientables. Cette solution, qui combine des éléments optiques à de la mécanique de précision, implique également un coût élevé.

Par ailleurs, lorsqu'elle traverse le faisceau lumineux, la cellule sanguine dévie la trajectoire des rayons lumineux. L'intensité et l'angle des rayons déviés permettent d'obtenir des informations sur le type de la cellule. Deux gammes d'angles sont généralement utilisées : de petits angles inférieurs à dix degrés par rapport à l'axe optique et de grands angles sensiblement perpendiculaires à l'axe optique. Dans la gamme des petits angles, deux informations sont utiles : les pertes dans l'axe et la diffraction. Perpendiculairement à l'axe optique, on mesure généralement la diffusion et la fluorescence. Pour les deux gammes d'angles, la lumière doit donc être répartie dans deux canaux différents. Ceci est généralement réalisé par des miroirs dichroïques ou encore par des filtres interférentiels. Ces composants optiques sont tous deux réalisés par dépôts de couches minces sur un substrat de verre. Leur efficacité est bonne mais il existe une grande disparité d'un filtre à l'autre et leur durée de vie est limitée. Ils doivent donc être changés régulièrement.

Tous ces dispositifs généralement encombrants, sont en outre fragiles et demandent un entretien, lui aussi très coûteux. De tels dispositifs sont donc réservés à des laboratoires d'analyses suffisamment importants pour pouvoir investir dans de tels automates.

La divulgation a pour but de proposer un dispositif de différenciation leucocytaire et/ou le comptage leucocytaire plus simple et plus économique tant à fabriquer qu'à entretenir, permettant l'utilisation d'automates, ainsi équipés, par des laboratoires de moindre importance, tout en gardant une qualité de mesure suffisante.

Dans la divulgation il est proposé un dispositif optique pour le comptage et/ou la différenciation leucocytaire dans un automate d'analyse sanguine, **caractérisé en ce qu'**il comprend une source lumineuse du type diode électroluminescente pour éclairer un échantillon sanguin qui circule dans la cuve optique selon un axe d'injection, à l'aide d'un faisceau lumineux source. Une telle diode permet d'obtenir un faisceau lumineux plus homogène sur la largeur de sa section, donc une zone de lecture plus large et homogène.

De préférence, la diode émet une lumière dont la longueur d'onde est inférieure à 600 nanomètres, et de façon encore plus préférée inférieure à 500 nanomètres. Une telle longueur d'onde permet un meilleur rendement de diffraction, donc une meilleure précision pour des mesures utilisant la diffraction.

En outre, la largeur du faisceau émis par le dispositif optique, c'est-à-dire le faisceau source qui éclaire le flux échantillon, est avantageusement comprise entre 50 et 200 microns (µm), au voisinage de l'axe d'injection, ce qui permet d'éclairer un flux échantillon plus large, tout en permettant une précision suffisante aux mesures effectuées. Encore plus avantageusement, cette largeur est comprise entre 90 et 120 microns. Une telle largeur de flux est notamment permise par l'utilisation de diodes électroluminescente.

De préférence, le faisceau lumineux source est émis sensiblement en direction de la cuve, sensiblement transversalement à la direction d'écoulement de l'échantillon. Une lame transparente prévue pour être traversée entre deux faces opposées par le faisceau source, montée en rotation et disposée entre la diode et la cuve peut permettre de déplacer transversalement le faisceau lumineux, grâce à sa double réfraction lors de la traversée de la lame. La rotation de la lame permet de modifier l'angle d'incidence du faisceau sur la lame, et ainsi de régler la valeur du décalage transversal. De préférence, la lame transparente est montée en rotation autour d'un axe sensiblement parallèle au déplacement de l'échantillon sanguin dans la cuve.

Au-delà de la cuve optique, on utilise avantageusement des moyens de séparation par pertes de Fresnel pour un faisceau lumineux résultant incident issu du faisceau lumineux source, séparant ainsi ledit faisceau en un faisceau résultant axial, et au moins un faisceau résultant de perte constitué par les pertes de Fresnel au passage des moyens de séparations. Les moyens de séparation comprennent au moins une surface de séparation qui est une surface d'un matériau de séparation transparent, le faisceau axial ayant traversé le matériau transparent et le faisceau issu des pertes de Fresnel ayant été réfléchi par la surface de séparation, ladite surface étant biaise relativement au faisceau lumineux au delà de la cuve. Une simple lame de verre, peu coûteuse, peut servir de moyens de séparation. En outre elle a une durée de vie quasiment illimitée et sans maintenance, contrairement aux miroirs dichroïques ou aux filtres interférentiels.

Le dispositif peut comprendre en outre un appareil pour mesurer la lumière du faisceau résultant axial et au moins un autre appareil pour mesurer la lumière de l'au moins un faisceau issu des pertes de Fresnel. Ces appareils de mesure peuvent notamment comprendre des moyens pour une mesure parmi une mesure de la fluorescence, une mesure des pertes lumineuses au voisinage de l'axe et une mesure de la diffraction au voisinage de l'axe. Il peut en outre comprendre des moyens de mesure de la diffraction du faisceau lumineux aux grands angles par l'échantillon dans la cuve. A titre d'exemple, ces grands angles peuvent être des angles compris entre 60° et 150°.

Le dispositif peut aussi comprendre, sur le trajet du faisceau avant la cuve, au moins un diaphragme d'arrêt pour une lumière parasite.

La divulgation porte aussi sur un appareil d'hématologie, notamment un automate d'analyse sanguine équipé d'un tel dispositif.

La présente divulgation vise également une cuve optique à circulation pour un dispositif optique adapté au comptage et à la différenciation leucocytaire, par exemple un cytomètre de flux, ainsi qu'un appareil d'analyse équipé d'une telle cuve. Le but de l'invention est de proposer une cuve plus simple et plus économique tant à fabriquer qu'à entretenir, permettant l'utilisation d'automates ainsi équipés par des laboratoires de moindre importance, tout en gardant une qualité de mesure suffisant.

Selon la divulgation, une cuve à circulation pour un dispositif optique pour le comptage et la différenciation leucocytaire dans un automate d'analyse sanguine, est **caractérisée en ce que** dans une zone d'analyse de la cuve (304), la section de la cuve a au moins une dimension transversale comprise entre 1 et 5 millimètres. Cette section peut être sensiblement rectangulaire et la dimension transversale être mesurée sur l'un et/ou l'autre des côtés du rectangle.

Une telle cuve peut alors être réalisée, au moins partiellement, en un matériau plastique injecté. Une telle cuve est particulièrement avantageuse à fabriquer relativement aux cuves de l'art antérieur, généralement formée de parois en quartz assemblées par collage.

La cuve peut en outre comprendre au moins une lentille moulée d'une seule pièce avec la cuve. Cette au moins une lentille peut comprendre une lentille prévue pour être disposée latéralement relativement à un axe optique. Elle peut comprendre une lentille hémisphérique.

La cuve peut comprendre selon un axe optique, une fenêtre pour l'entrée d'un faisceau lumineux et une fenêtre pour la sortie du faisceau. Au moins une fenêtre peut être moulée d'une seule pièce avec cuve et/ou être un insert en une matière transparente, par exemple du quartz ou du verre.

La cuve peut avantageusement comprendre un injecteur pour un flux d'échantillon et des moyens pour former un flux de manchonnage autour du flux d'injection. L'injecteur peut comprendre un orifice de sortie dont le diamètre est compris entre 20 microns et 150 microns, permettant d'obtenir un flux d'échantillon sensiblement plus large que les flux de l'art antérieur. Contrairement aux dispositifs de l'art antérieur, ce n'est pas le flux de manchonnage qui impose la largeur du flux échantillon en l'étirant, mais la forme et la section de sortie de l'injecteur. Le flux de gainage n'a donc plus un rôle actif, mais seulement un rôle passif, notamment, par exemple, pour centrer le flux échantillon dans une cuve de grande largeur.

Selon un premier mode de réalisation, cet injecteur peut être formé d'une seule pièce en un matériau sensiblement rigide. Ce matériau peut être, par exemple, un acier inoxydable, une céramique, du rubis synthétique ou une matière plastique ou plusieurs de ces matériaux.

Selon un deuxième mode de réalisation cet injecteur peut comprendre un tube de structure rigide, par exemple métallique, par exemple en acier inoxydable, et à l'intérieur du tube de structure, un tube de gainage en matériau plastique prolongé par un bec formé d'une seule pièce avec le tube de gainage. Le matériau plastique de l'injecteur peut être un polytétrafluoroéthylène, qui permet une circulation plus aisée de l'échantillon dans la tube et en réduit les risques l'encrassement.

La divulgation porte en outre sur un injecteur pour une cuve selon la divulgation, injecteur réalisé selon l'un de ces modes de réalisation.

La divulgation porte aussi sur un appareil d'hématologie, notamment un automate d'analyse sanguine, équipé d'une cuve selon l'invention.

La présente divulgation vise également un dispositif hydraulique pour un appareil d'analyse hématologique, qui soit simplifié et plus économique tant à fabriquer qu'à entretenir et qui permette l'utilisation d'automates équipés d'un tel dispositif par des laboratoires de moindre importance, tout en gardant une qualité de mesure suffisante. La présente divulgation vise aussi un procédé d'analyse adapté à un tel dispositif.

La présente divulgation propose ainsi un dispositif hydraulique pour un appareil d'analyse sanguine, notamment un automate, comprenant des moyens pour injecter sous pression un flux d'échantillon dans une cuve optique à circulation et pour créer un flux de manchonnage liquide autour du flux d'échantillon, avec un liquide de manchonnage, **caractérisé en ce qu'**il comprend des moyens pour réguler un débit du flux d'échantillon relativement au débit du liquide de manchonnage. Une telle régulation peut permettre de maintenir des flux homogènes et sensiblement non turbulents dans la cuve.

Les moyens d'injection peuvent comprendre des seringues, un circuit hydraulique et des électrovannes. Ces moyens peuvent comprendre des moyens pour injecter l'échantillon sous pression relativement au flux de manchonnage.

Ce dispositif peut avantageusement comprendre des moyens pour former un piston pour l'échantillon injecté avec un liquide de pistonnage. Un tel liquide de pistonnage permet de n'utiliser qu'un petit échantillon suffisant pour l'analyse, le reste du liquide nécessaire pour l'injection étant un liquide disponible dans l'appareil d'analyse, et moins précieux que l'échantillon.

Le manchonnage est particulièrement avantageux pour utiliser une cuve de grande section tout en maintenant une section réduite pour le flux d'échantillon. Parmi les moyens de régulation du flux d'échantillon relativement au flux de manchonnage, le dispositif peut avantageusement comprendre des moyens pour réguler un débit du liquide de pistonnage relativement au débit du liquide de manchonnage. Les moyens de régulation peuvent comprendre des moyens de perte de charge dans un circuit de dérivation pour le liquide de pistonnage et/ou des moyens de perte de charge dans un circuit de dérivation pour le liquide de manchonnage. Par exemple, les moyens de perte de charge peuvent êtres choisis parmi une longueur connue d'un tuyau calibré, une résistance hydraulique fixe et une résistance variable.

Le dispositif hydraulique peut ne comprendre qu'une seule motorisation, par exemple un seul moteur électrique, pour générer simultanément le flux d'échantillon et le flux de manchonnage. En outre, il peut comprendre au moins deux seringues pour générer le flux d'échantillon et le flux de manchonnage, les pistons des seringues étant rigidement liés entre eux. Ils sont ainsi animés d'un mouvement commun et les flux d'échantillon et de manchonnage sont bien simultanés.

Notamment, une cuve à hydrofocus de l'art antérieur peut être utilisée avec un circuit tel que précédemment décrit, l'injection de l'échantillon dans cette cuve pouvant se faire sans pression relativement au fluide de manchonnage.

Selon la divulgation, il est aussi proposé un procédé d'analyse d'un échantillon sanguin dans un cytomètre à circulation, **caractérisé en ce que** l'on injecte, éventuellement sous pression, un échantillon sanguin dans une cuve à circulation du cytomètre, l'échantillon y formant un flux d'échantillon et on crée un flux de manchonnage liquide autour du flux d'échantillon, avec un liquide de manchonnage, **caractérisé en ce qu'**on régule le débit du flux d'échantillon relativement au débit du liquide de manchonnage.

En particulier, on peut introduire l'échantillon dans une branche d'injection d'un circuit hydraulique, et introduire en amont de l'échantillon dans la branche d'injection, un liquide de pistonnage, le liquide de pistonnage servant à pousser l'échantillon lors de son injection dans la cuve. Ce liquide de pistonnage peut être choisi parmi un réactif et un diluant, de préférence un réactif. Il n'est donc pas utile de prévoir un autre liquide que ceux strictement nécessaires pour la préparation de l'échantillon en vue de son ou de ses analyses.

On peut aussi créer autour du flux d'échantillon dans la cuve, un flux de manchonnage avec un liquide de manchonnage. Ce liquide de manchonnage peut aussi être choisi parmi un réactif et un diluant, de préférence un diluant. Là aussi, Il n'est donc pas utile de prévoir un autre liquide que ceux strictement nécessaires pour la préparation de l'échantillon en vue de son ou de ses analyse.

Dans le cas de mise en oeuvre d'un procédé d'hydrofocus, ou d'une cuve, il est avantageux de réguler le débit du liquide de pistonnage relativement au débit du liquide de manchonnage, par exemple en introduisant une perte de charge dans un circuit de dérivation pour un liquide de pistonnage et/ou des moyens de perte de charge dans un circuit de dérivation pour un liquide de manchonnage.

Dans un procédé utilisant une cuve, on peut aisément prévoir que l'échantillon du sang présente un taux de dilution d'au moins 1/100ème. En effet, dans un tel procédé, on peut introduire l'échantillon sous pression relativement au fluide de manchonnage, dans la cuve, à une vitesse supérieure à celle des procédés de l'art antérieur, et avec des largeurs de sections supérieures pour le flux d'échantillon dans la cuve. Ainsi, sans augmenter le temps d'analyse, on peut pour une différenciation et un comptage des globules blancs utiliser un taux de dilution identique à celui pratiqué habituellement pour la mesure de l'hémoglobine, en particulier des taux de dilution sensiblement compris entre 1/100^{ème} et 1/500^{ème}, particulièrement entre 1/160^{ème} et 1/180^{ème}.

La divulgation concerne aussi un appareil d'hématologie, notamment un automate d'analyse sanguine, **caractérisé en ce qu'**il comprend un dispositif hydraulique selon l'invention.

La présente invention sera mieux comprise et d'autres avantages apparaîtront à la lumière de la description qui va suivre d'exemples de réalisation, description faite notamment en référence aux dessins annexés sur lesquels :
- la figure 1 illustre de manière schématique un exemple d'appareillage ;
- les figures 2a-2e sont des graphes de test de linéarité de la mesure de l'hémoglobine par spectrophotométrie selon le procédé de l'invention;
- les figures 2f-2i sont des cytographes correspondants ;
- la figure 3 est une vue schématique d'un automate d'analyse d'un échantillon de sang mettant en oeuvre un dispositif hydraulique ;
- la figure 4 est une vue schématique longitudinale d'ensemble d'un dispositif optique ;
- la figure 5 est une vue schématique longitudinale plus détaillée du dispositif optique de la figure 4, dans un plan perpendiculaire à celui de la figure 4 ;
- la figure 6 est une vue en perspective d'une cuve optique ;
- la figure 7 est une vue en coupe longitudinale d'un premier mode de réalisation d'un injecteur pour une cuve optique ;
- la figure 8 est une vue en coupe longitudinale d'un deuxième mode de réalisation d'un injecteur pour une cuve optique ;
- la figure 9 est une vue en coupe longitudinale d'une extrémité de l'injecteur de la figure 8 ;
- la figure 10 est une vue en coupe longitudinale d'une cuve illustrant un procédé d'injection de l'art antérieur pour l'échantillon de sang dans la cuve ; et
- la figures 11a-11c sont des graphes illustrant les résultats obtenus avec un automate mettant en oeuvre le procédé de l'invention et utilisant un cytographe avec le dispositif et la cuve optiques.

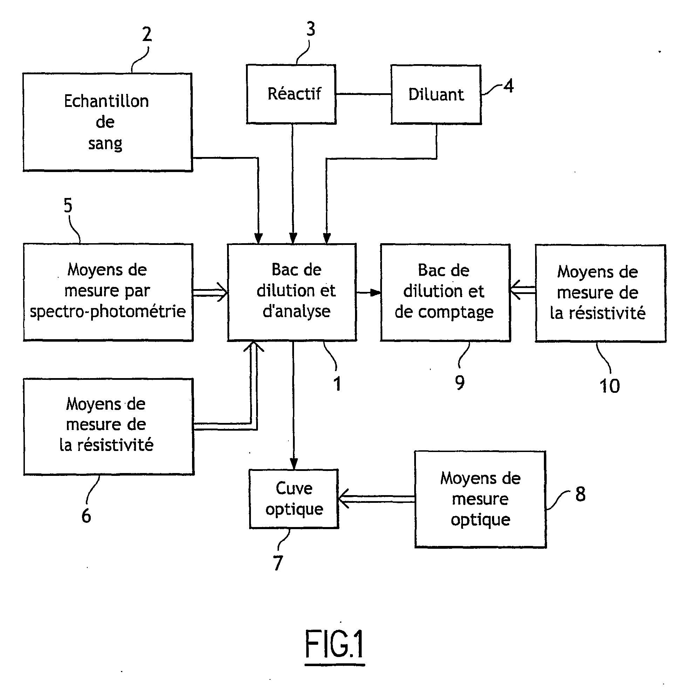

Sur la figure 1, est schématisé un bac unique 1 de dilution et d'analyse pouvant être alimenté par un échantillon de sang 2 à analyser, un diluant 3 et un réactif 4 formant ensemble une solution d'analyse. Ce bac 1 est équipé de moyens de mesure par photométrie 5 du taux d'hémoglobine dans ladite solution d'analyse et de moyens de mesure 6 de la résistivité de ladite solution d'analyse pour compter le nombre global de globules blancs. Des moyens sont également prévus pour prélever une fraction de la solution d'analyse dans le bac d'analyse 1 et pour l'injecter dans une cuve optique 7 munie de moyens de mesure optique 8 (par exemple un cytomètre de flux) pour une analyse des globules blancs. Selon l'exemple choisi, des moyens sont en outre prévus pour prélever une fraction d'une pré-solution constituée de l'échantillon de sang et de diluant, et l'introduire dans un bac de comptage et de dilution 9 muni de moyens de mesure de la résistivité 10 de ladite fraction pour compter les globules rouges et les plaquettes. De manière classique, l'appareillage est muni de moyens de chauffage pour obtenir une température thermostatée sensiblement à 35°C. Cette température permet un temps de la réaction de lyse et une qualité de lyse des globules rouges optimale.

L'appareillage fonctionne de la manière suivante :
- une aliquote de sang (15,6 µl) est injectée dans la cuve d'analyse 1 et diluée avec 2 ml de diluant de manière à former une pré-solution d'analyse ; le taux de dilution est de 1/130^{e} ;
- une fraction très faible (environ 20 µl) est prélevée sur cette pré-solution d'analyse et déposée dans le bac 9 de comptage des globules rouges et des plaquettes ;
- 0,7 ml de réactif est alors ajouté au reliquat dans le bac d'analyse 1 : la lyse dure environ 10 secondes (pour détruire les globules rouges, former et stabiliser le complexe oxyhémoglobine), la solution d'analyse ainsi formée présente un taux de dilution final d'environ 1/173^{e} ; une fraction de ladite solution d'analyse est prélevée et injectée dans la cuve optique 7 où peut avoir lieu l'analyse des globules blancs (comptage et/ou différenciation leucocytaires par sous populations) ; simultanément dans le bac d'analyse 1, les globules blancs sont comptés par une mesure de résistivité et l'hémoglobine par une mesure par absorbance à la longueur d'onde du complexe oxyhémoglobine formé.

Un dispositif optique, particulièrement adapté à une analyse leucocytaire sur une solution d'analyse ayant un taux de dilution inférieur à 1/100^{e} est décrit plus loin, plus particulièrement adapté à une dilution comprise entre 1/160^{e} et 1/180^{e}. Par convention, on considère qu'un taux de dilution de 1/160^{e} est inférieur à un taux de 1/100^{e}.

Il va de soi que des variantes de réalisation du procédé et de l'appareillage décris ci-dessus sont possibles :
- pour l'appareillage : on peut prévoir des moyens pour introduire séparément le composé de lyse, le composé leucoprotecteur et le composé stabilisateur du complexe formé avec l'hémoglobine dans la cuve d'analyse 1, et donc plus sous la forme d'un mono-réactif ; les moyens 6 pour mesurer la résistivité de la solution d'analyse sont optionnels, le nombre global de globules blancs pouvant être obtenus par l'analyse optique de la solution d'analyse ; de même pour le bac de comptage 9 et les moyens de mesure 10 de la résistivité dans ce bac peuvent être prévus uniquement si l'on souhaite une analyse complète de l'échantillon de sang ;
- de même pour le procédé : on peut prévoir l'introduction des composés réactionnels de manière indépendante ou groupée à la place d'un mono-réactif, l'introduction pouvant être menée de manière simultanée ou successivement ; on peut omettre l'étape préalable de comptage des globules rouges et des plaquettes et l'étape de comptage global des globules blancs ; par ailleurs, on peut réaliser deux dilutions successives de l'échantillon de sang : une première dilution particulièrement adaptée à une différenciation leucocytaire (environ au 1/80^{e}) telle qu'elle est menée dans les cytomètres classiques connus de type hydrofocus, sur laquelle est prélevée la fraction nécessaire à cette différenciation leucocytaire, puis dans un second temps une deuxième dilution adaptée à une mesure de l'hémoglobine (comprise entre 1/100^{e} et 1/500^{e}) telle qu'elle est possible avec les spectromètres connus.

Selon une autre variante encore, le bac 1 peut servir dans un second temps à faire le comptage des globules rouges et des plaquettes après nettoyage, en remplissant le bac avec un prélèvement en attente dans une aiguille d'une seringue.

On va maintenant décrire les résultats obtenus avec un exemple particulier de (mono)réactif :
Un monoréactif est préparé à partir de la formulation Eosinofix® de la société ABX commercialisée pour la détermination leucocytaire en cytométrie de flux et contenant à cet effet un composé pour lyser les globules rouges et un composé leucoprotecteur (cf le brevet EP0430750 d'ABX). Selon l'invention, on a ajouté à cette formulation un composé stabilisateur du complexe de l'hémoglobine.

### Mesure de l'hémoglobine par spectrophotométrie :

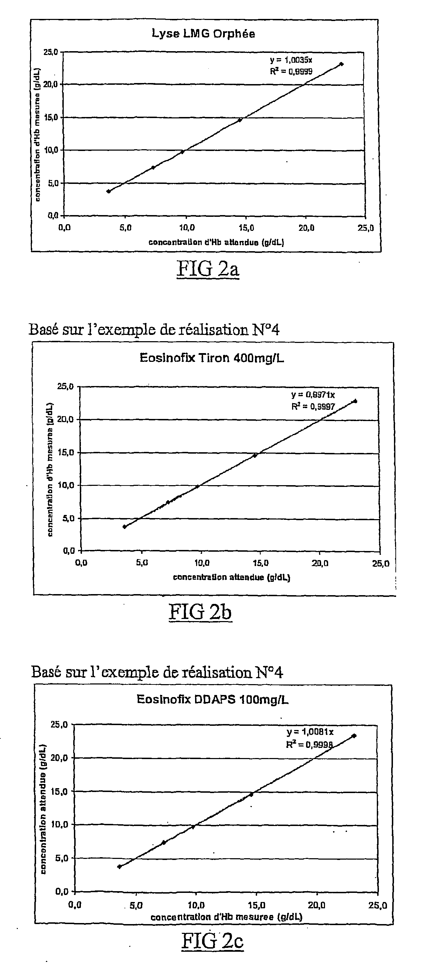

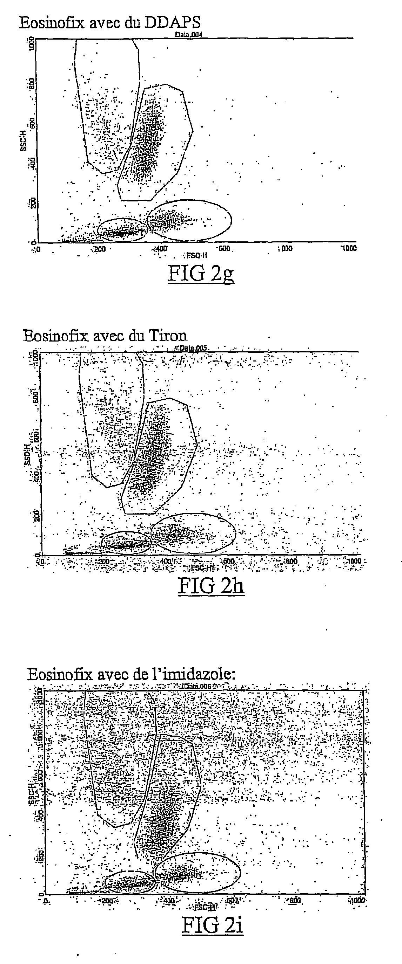

Des tests de linéarité ont été menés à l'aide d'un spectrophotomètre à 542nm. Les graphes sont donnés aux figures 2a-2e. Ils représentent les concentrations d'hémoglobine mesurées par rapport aux concentrations attendues. Plus précisément :
- la figure 2a correspond à une lyse de référence pour la mesure de l'hémoglobine par spectrophotométrie (LMG® vendue par la société ORPHEE);

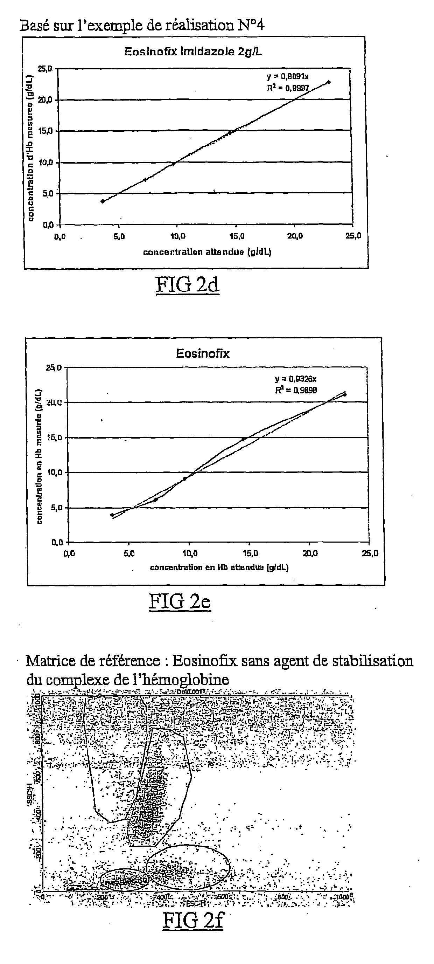

- les figures 2b, 2c et 2d correspondent au monoréactif selon l'exemple de réalisation N°4 avec respectivement comme agent de stabilisation du complexe de l'hémoglobine le Tiron, le DDAPS et l'imidazole ; et
- la figure 2e correspond au procédé de l'invention mis en oeuvre en utilisant comme monoréactif de l'éosinofix® seul, c'est-à-dire ne contenant pas d'agent de stabilisation du complexe de l'hémoglobine conformément à la présente invention.

Pour les trois essais effectués selon l'invention, on obtient bien un test de linéarité positif avec pour chacun un coefficient de corrélation R² de 1 ± 10⁻⁴ (reporté sur la figure). Ce résultat est conforme à celui obtenu avec la lyse de référence de la figure 2a. Cela signifie que le procédé de l'invention permet bien de mesurer un taux réel d'hémoglobine dans un échantillon de sang.

En revanche, comme on le voit sur la figure 2e, avec le réactif (Eosinofix) sans stabilisateur de l'hémoglobine, on n'obtient pas une relation linéaire. Cela signifie que ce réactif seul ne peut être utilisé pour mesurer un taux d'hémoglobine

### Différentiation leucocytaire par cytométrie de flux

Les figures 2f à 2i sont des cytographes obtenus à l'aide d'un cytomètre de flux BD FACScan®,, correspondant respectivement à l'Eosinofix seul, l'Eosinofix auquel on ajouté du DDAPS, du Tiron et de l'imidazole. Sur ces figures, on voit que la différentiation des sous-populations de globules blancs est bien réalisée et de manière comparable à un réactif classique de différentiation leucocytaire (matrice obtenue avec l'Eosinofix à la figure 2f).

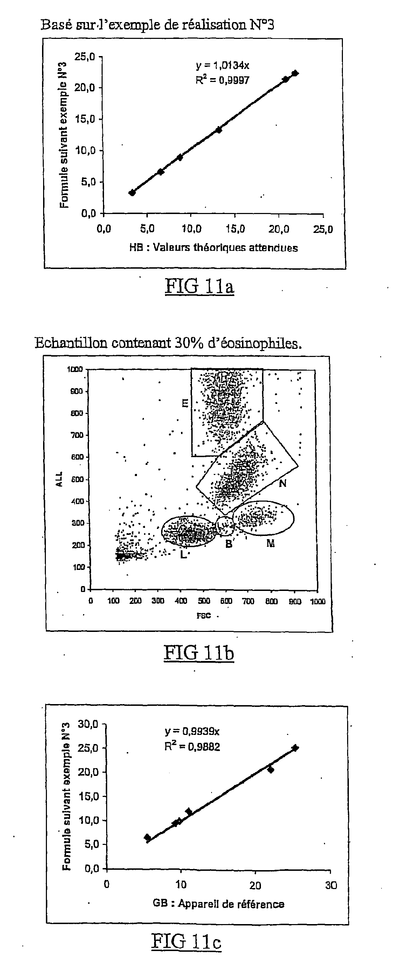

On peut aussi se référer au cytographe de la figure 11b (décrite plus loin) obtenu notamment avec un cytomètre.

On va maintenant décrire le dispositif hydraulique.

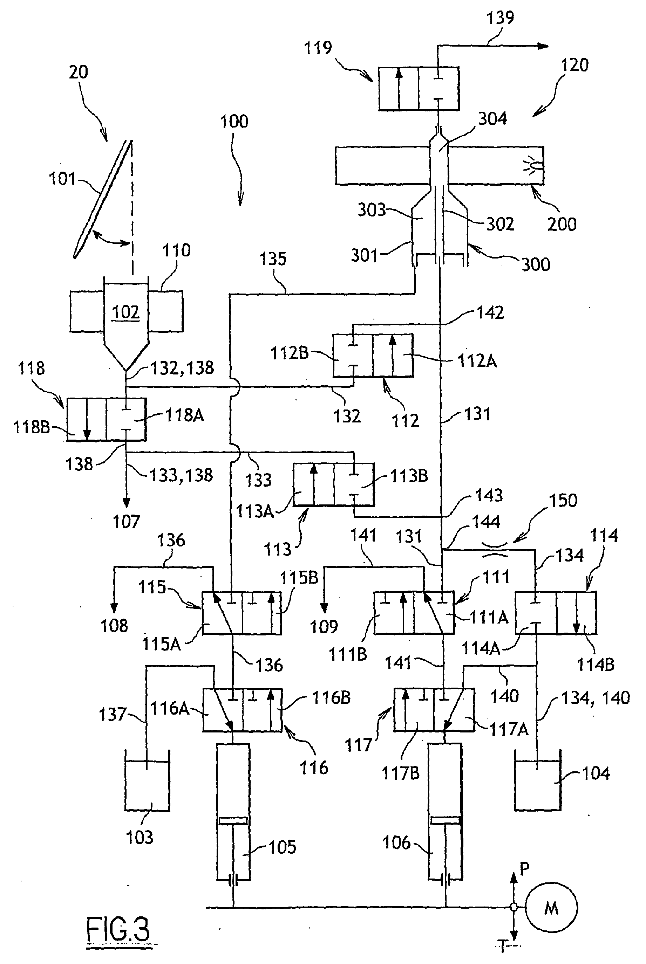

La figure 3 représente partiellement le schéma d'un circuit hydraulique 100 et quelques équipements d'un automate 20 d'analyses sanguines, pour autant qu'ils permettent la compréhension du dispositif hydraulique.

L'automate illustré à la figure 3 comprend notamment une aiguille 101 pour prélever du sang à analyser dans un tube ayant servi à son stockage et à son transport jusqu'à l'automate. Le sang prélevé est versé sous forme d'un échantillon par l'aiguille dans un bac 102. Le bac 102 est notamment prévu pour la dilution et/ou la lyse des globules rouge de l'échantillon de sang. Tout ou partie de l'échantillon, avant ou après dilution, peut être prélevé en vu d'analyses dans une autre partie de l'automate, par exemple dans un dispositif 120, décrit ultérieurement. Un dispositif d'analyse de l'hémoglobine 110 (un spectrophotomètre par exemple) est disposé au voisinage du bac 102. Une réserve 103 pour un produit de dilution et une réserve 104 pour un réactif, notamment de lyse sont reliées au bac 102 via le circuit hydraulique 100.

Un autre dispositif d'analyse 120 est plus particulièrement dédié au comptage et à la différenciation des globules blancs, par exemple sur le tout ou la partie de l'échantillon prélevé dans le bac 102. Par la suite nous appèlerons aussi échantillon ce tout ou cette partie. Le dispositif d'analyse des globules blancs 120 comprend notamment un dispositif optique 200 et une cuve optique 300. La cuve optique est reliée au bac 102 via le circuit hydraulique 100.

Un jeu de seringue permet le déplacement des liquides dans le circuit hydraulique. Parmi ces seringues, une seringue 105 dédiée au diluant et une seringue 106 dédiée au réactif sont représentées pour la bonne compréhension du dispositif. D'autres seringues qui ne sont pas représentées car elles ne sont pas nécessaires à la compréhension du dispositif, peuvent compléter le dispositif.

Outre des conduites pour la circulation des liquides, le circuit hydraulique comprend des électrovannes pour la commutation de différents circuits dans le circuit hydraulique 100, selon l'utilisation qui en est faite à un moment donné de l'analyse. Huit électrovannes 111-119 parmi les électrovannes du circuit hydraulique 100 sont illustrées à la figure 3. Chaque électrovanne comprend deux positions, chacune repérée respectivement par la lettre A ou B.

La conception du circuit hydraulique tel qu'il sera décrit par la suite, permet de n'utiliser qu'une seule motorisation M pour les seringues illustrées. La même motorisation peut d'ailleurs être utilisée pour d'autres seringues. Ainsi, les pistons des seringues 105,106 sont rigidement reliés entre eux. Ils ont donc un mouvement simultané, soit en poussée P, lorsqu'ils s'enfoncent dans le cylindre respectif de chaque seringue, ou en traction T lorsqu'ils en sont extraits.

On va maintenant décrire la disposition puis le fonctionnement hydraulique de l'automate.

La cuve 300 comprend un corps extérieur 301 et un injecteur 302, à l'intérieur du corps 301, un volume de manchonnage 303 est formé entre le corps et l'injecteur.

Le circuit hydraulique 100 comprend :
- une branche d'injection 131 qui s'étend en amont de l'injecteur, entre l'injecteur et la vanne 111 ;
- une branche d'échantillon 132 qui se raccorde en un branchement d'échantillon 142 sur la branche d'injection et s'étend jusqu'au bac 102 ;
- une branche d'aspiration 133 qui se raccorde en un branchement d'aspiration 143 sur la branche d'injection, en amont du branchement d'échantillon 142, via la vanne 113 et s'étend jusqu'à une source de vide 107, par exemple une seringue ou une pompe péristaltique ;
- une branche de décharge 134 qui se raccorde en un branchement de décharge 144 sur la branche d'injection, en amont du branchement d'aspiration 143, et s'étend jusqu'à la réserve de produit de réactif 104 ;
- une branche de manchonnage 135 qui s'étend en amont du corps 301 et relie le volume de manchonnage et la vanne 115 ;
- une branche de dilution 136 qui s'étend entre la vanne 116 et une utilisation 108 pour le diluant via la vanne 115;
- une branche de diluant 137 qui s'étend entre la réserve de diluant 103 et la vanne 116 ;
- une branche de réactif 140 qui s'étend entre la réserve de réactif 104 et la vanne 117 ;
- une branche de réaction 141 qui s'étend entre la vanne 117 et une utilisation 109 pour le réactif via la vanne 111;
- une branche de vidange 138 pour le bac 102 qui s'étend entre le bac 102 et la source de vide 107 via la vanne 118, la branche d'échantillon 132 étant reliée avec la branche de vidange entre le bac 102 et la vanne 118, et la branche d'aspiration étant reliée avec la branche de vidange 132 au-delà de la vanne 118 relativement au bac; et,
- une branche de sortie 139 qui relie l'aval de la cuve 300 , via la vanne 119, avec un bac à déchet, par exemple à la pression atmosphérique où via une source d'aspiration, une seringue ou une pompe péristaltique.

Dans une première position 116A de la vanne 116, la seringue de dilution 105 est en communication avec la réserve de diluant, de sorte qu'une traction T permet de remplir la seringue 105 avec du diluant.

La seringue de dilution contenant du diluant, dans un premier cas, la vanne 116 étant dans sa deuxième position 116B qui relie la seringue 105 avec la branche de dilution 136 et la vanne 115 étant dans une première position 115A qui relie la branche de dilution avec l'utilisation 108 pour le diluant, une poussée P permet de déplacer le diluant jusqu'à cette utilisation 108, par exemple dans le bac 102, par exemple pour une dilution de l'échantillon global.

Dans un deuxième cas, la vanne 116 étant dans sa deuxième position 116B et la vanne 115 étant dans sa deuxième position 115B qui relie la branche de dilution avec la branche de manchonnage 135, une poussée P permet de déplacer le diluant jusque dans la cuve optique 300, pour y former un flux de manchonnage. L'utilité de ce flux de manchonnage dans le cadre de l'invention sera analysée lors d'une description ultérieure de la cuve 300.

La vanne 117 étant dans une première position 117A qui relie la seringue de réactif avec la réserve 104 de réactif, et la vanne 114 étant dans une première position 114A qui coupe la branche de décharge 134, une traction T permet de remplir la seringue de réactif 106 avec du réactif.

La seringue de réactif contenant du réactif, dans un premier cas, la vanne 117 étant dans sa deuxième position 117B qui relie la seringue de réactif 104 avec la branche réaction 141 et la vanne 111 étant dans une première position 111A qui relie la branche de réaction avec l'utilisation 109 pour le réactif, une poussée P permet de déplacer le réactif jusqu'à cette utilisation 109, par exemple dans le bac 102, par exemple pour une lyse de l'échantillon global.

Dans un deuxième cas, la vanne 117 étant dans sa deuxième position 117B et la vanne 111 étant dans sa deuxième position 111B qui relie la branche de réaction 141 avec la branche de d'injection 131, la seringue de réactif 106 est directement reliée à l'injecteur 302.

La vanne 118 étant dans une première position 118A qui isole la branche d'aspiration 133 d'avec la branche d'échantillon 132 au travers de la branche de vidange, la vanne 112 étant dans une première position 112A qui relie l'amont à l'aval de la branche d'échantillon 132, la vanne 113 étant dans une première position 113A qui relie l'aval à l'amont de la branche d'aspiration 133, donc à la source de vide 107, l'échantillon à analyser est aspiré jusque dans la branche d'injection 131, entre le branchement d'échantillon 142 et le branchement d'aspiration 143.

La branche de décharge 134 comprend une résistance fluidique 150 variable ou calibrée.

Lorsque la seringue de diluant 105 contient du diluant, que la seringue de réactif 106 contient du réactif et qu'un échantillon sanguin à analyser se trouve dans la branche d'injection 131 ; lorsqu'en outre les vannes 112,113 sont dans leurs deuxièmes positions 112B,113B qui isolent l'amont et l'aval de leurs branches respectives ; lorsqu'encore les vannes 115,116 sont dans leurs deuxièmes positions 115B,116B qui relient la seringue 105 de diluant avec le volume de manchonnage 303; lorsqu'enfin les vannes 111,117 sont dans leurs deuxièmes positions 111B,117B qui relient la seringue de réactif 106 avec l'injecteur 302 et que la vanne 114 est dans sa deuxième position 114B ; un seul mouvement de poussée P généré par la seule motorisation M, permet de propulser le diluant, le réactif et l'échantillon de sang en direction et au travers de la cuve 300, alors qu'une partie du réactif, fonction de la résistance fluidique 150, est retournée dans la réserve de réactif 104.

La résistance 150 permet notamment de régler les débits relatifs des liquides de manchonnage et de pistonnage. Cela permet d'adapter ces débits aux différentes fonctions de ces liquides. En particulier, cela permet d'avoir des vitesses d'écoulement similaires pour le manchonnage et l'échantillon dans la zone d'analyse 304 lorsque l'on utilise une cuve à hydrofocus classique.

Notamment, la branche de décharge 134 et les dispositions précédemment décrites permettent l'utilisation d'une unique motorisation, donc de réduire notablement le coût d'un automate d'analyse, ainsi que son encombrement.

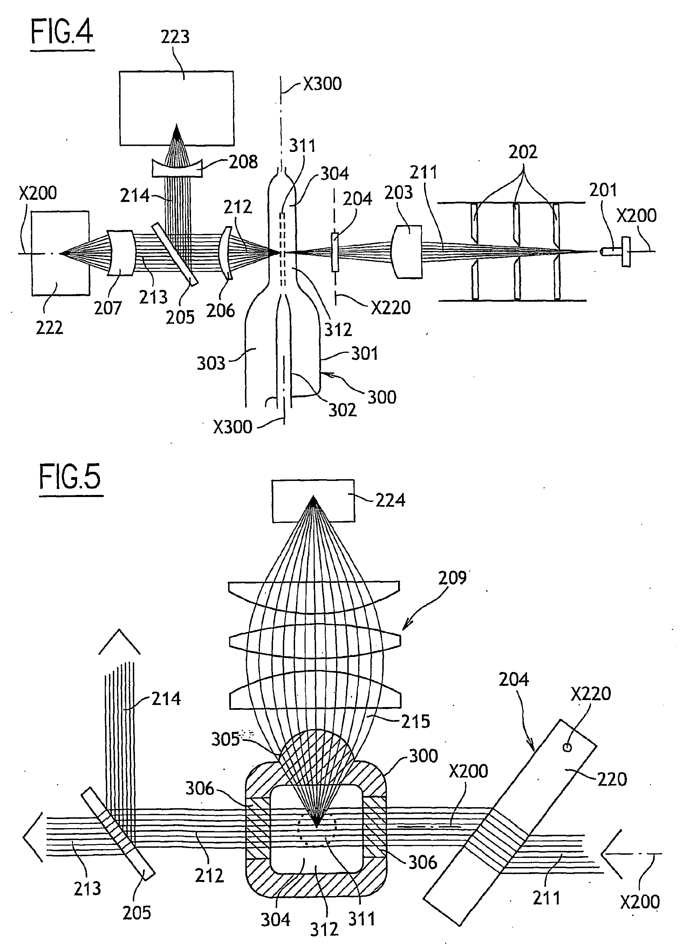

Le diluant forme dans une zone d'analyse 304 de la cuve 300 un flux de manchonnage pour l'échantillon (voir particulièrement les figures 4 et 5). Le réactif, situé en amont de l'échantillon dans la branche d'injection 131, sert de liquide de pistonnage, c'est-à-dire qu'il permet de transmettre le mouvement du piston de la seringue de réactif à l'échantillon. Ainsi, il n'est pas utile de remplir la seringue de réactif avec l'échantillon pour pouvoir en faire l'analyse. Ainsi, même un échantillon de faible volume peut être analysé, et la totalité de cet échantillon peut être injecté et analysé sans qu'une partie ne reste dans la branche d'injection 131 ou dans la seringue 106.

Bien sûr, d'autres seringues, vannes et branches, non représentées à la figure 3, peuvent compléter le circuit hydraulique 100, pour le bon et complet fonctionnement de l'automate d'analyse 1.

On va maintenant décrire le dispositif optique 200, particulièrement en regard des figures 4 et 5.

Le dispositif optique comprend une source 201 de lumière sensiblement monochrome. Cette source de lumière est une diode électroluminescente. La lumière est émise principalement selon un axe optique X200. L'axe optique X200 est disposé sensiblement perpendiculairement à un axe d'injection X300 de déplacement de l'échantillon dans la cuve optique 300. Les deux axes X200 et X300 définissent ensemble un plan optique.

Pour éviter que le faisceau lumineux source 311 issus de la source 201 ne soit pas pollué par des lumières parasites, un jeu de trois diaphragmes est disposé chacun perpendiculairement sur le parcours du faisceau. Les diaphragmes 202 sont percés d'orifices dont le diamètre est sensiblement égal au faisceau et progressivement augmenté dans chaque diaphragme pour s'adapter au diamètre du faisceau à mesure où ce diamètre augmente en s'éloignant de la source 201. Le faisceau traverse ensuite un dispositif de focalisation 203 constitué par une ou plusieurs lentilles.

Au-delà du dispositif de focalisation le faisceau rencontre un dispositif de réglage qui permet de déplacer l'axe optique dans un plan perpendiculaire à l'axe d'injection X300, c'est-à-dire transversalement relativement au déplacement de l'échantillon dans la cuve. Un décalage latéral du faisceau peut conduire à un éclairage partiel ou nul de l'échantillon ce qui a une influence directe sur le résultat de l'analyse.

Dans le cadre de l'exemple décrit, le dispositif de réglage est constitué d'une lame transparente 220 montée en rotation autour d'un axe X220. L'axe 221 est sensiblement parallèle à l'axe d'injection X300. Si la lame est disposée perpendiculairement à l'axe optique X200, le faisceau la traverse sans être dévié. Par contre, si la lame forme un angle avec l'axe optique, une double réfaction, à l'entrée et à la sortie de la lame, décale le faisceau dans un plan perpendiculaire à l'axe de réglage X220. L'axe de réglage X220 étant sensiblement parallèle à l'axe d'injection X300, seul un décalage transversal est généré par la réfraction dans la lame. Le décalage est d'autant plus grand que l'épaisseur et/ou l'indice de réfraction de la lame sont grands et que la lame est inclinée relativement à l'axe optique. Ainsi, pour une lame d'épaisseur et d'indice de réfraction choisis, il suffit de faire tourner la lame 220 autour de son axe X220 pour régler la position du faisceau relativement à l'échantillon qui se déplace dans la zone d'analyse 304 de la cuve optique 300. Un tel dispositif de réglage est particulièrement économique relativement aux dispositifs de l'art antérieur, d'autant qu'une rotation précise est généralement plus facile à mettre en oeuvre qu'une translation précise, utilisant de la mécanique de grande précision.

Après avoir pénétré la cuve et traversé l'échantillon, le faisceau source 211 devient au moins partiellement un faisceau résultant axial 212, qui sort de la cuve sensiblement selon l'axe optique. Le faisceau résultant axial 212 est porteur d'informations sur l'échantillon qu'il vient de traverser.

Pour permettre des mesures simultanées de plusieurs de ces informations on doit pouvoir analyser le faisceau avec plusieurs appareils de mesure 222,223. En particulier, l'analyse optique repose sur la détection de la lumière diffractée selon deux gammes d'angles : petits et grands angles. Dans chacune des gammes d'angles, on utilise deux informations différentes. Il convient donc de répartir la lumière dans deux canaux différents pour chaque gamme. On utilise donc des moyens 205 pour séparer le faisceau résultant 212 en deux faisceaux résultants 213,214. Les moyens de séparation sont principalement constitués d'une lame séparatrice 205. Cette lame séparatrice est une lame transparente en verre. Elle est disposée à 45 degrés de l'axe optique. Il en résulte un faisceau résultant axial secondaire 213, formé par la lumière ayant traversé la lame séparatrice, et un faisceau résultant de perte 214 formé par les pertes de Fresnel, c'est-à-dire par la lumière réfléchie par la lame séparatrice.

Une telle lame séparatrice est d'un coût très faible relativement aux moyens de séparation utilisés dans l'art antérieur dans les dispositifs d'analyse optique de ce type. En particulier, du fait qu'elle ne comprend aucun revêtement réfléchissant ajouté, elle est quasiment inaltérable dans le temps et ne nécessite sensiblement aucun entretien. Compte tenu des réflexions multiples à l'intérieur de la lame et tenant compte de la polarisation du rayonnement incident du faisceau résultant axial, entre 5 et 15% de l'énergie est réfléchie, le reste étant transmis sous la forme du faisceau résultant axial secondaire.

Entre la cuve et la lame séparatrice, le faisceau résultant axial 212 est rendu parallèle par des moyens adaptés 206. Au-delà de la lame séparatrice, les faisceaux résultants 213,214 sont de nouveau focalisés par des moyens adaptés respectifs 207,208, en vue de leur analyse par les appareils de mesure respectifs 222,223.

Dans l'exemple décrit, l'appareil de mesure 222, qui analyse le faisceau résultant axial secondaire 213 est un appareil de mesure de la diffraction au voisinage de l'axe optique par les cellules sanguines (dite mesure du FSC). Dans l'exemple décrit, l'appareil de mesure 223, qui analyse le faisceau issu des pertes de Fresnel 214 est un appareil de mesure des pertes lumineuses dans l'axe (dite mesure de l'ALL), c'est-à-dire de l'occultation de la lumière par les cellules dans l'échantillon.

La figure 5 représente schématiquement une coupe de la cuve dans un plan perpendiculaire à l'axe d'injection X300 et contenant l'axe optique X200. Comme particulièrement illustré à cette figure, on analyse aussi la lumière réémise latéralement par l'échantillon en un flux résultant latéral 315, focalisé, au-delà de la cuve dans un appareil de mesure 224.

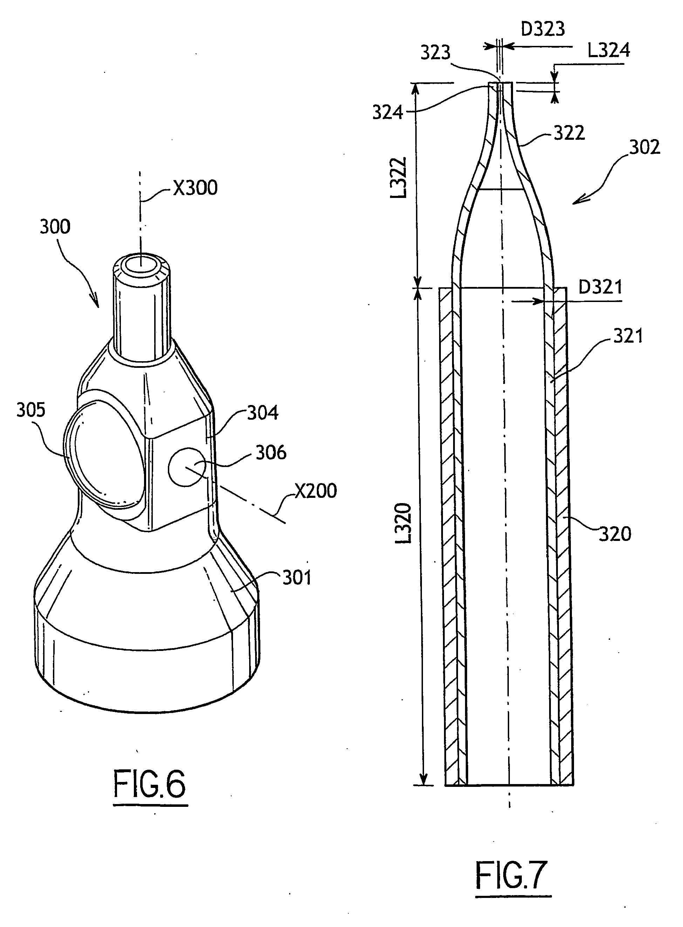

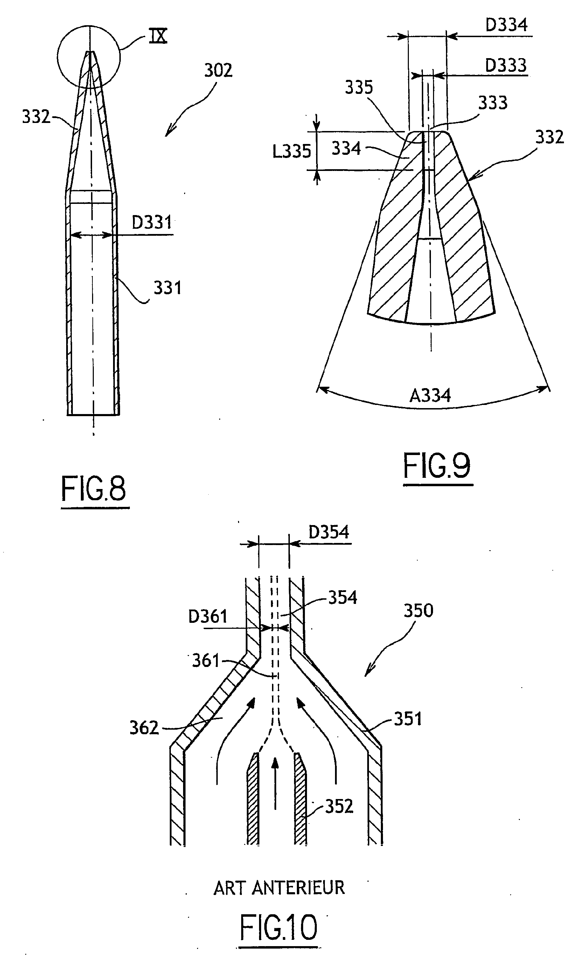

Notamment en référence à la figure 6, on va maintenant décrire une cuve optique, prévue notamment pour être utilisée avec un circuit hydraulique tel que décrit précédemment. Le fonctionnement de cette cuve peut être comparé à un fonctionnement de type hydrofocus de l'art antérieur, représenté très schématiquement à la figure 10.

La cuve 350 de la figure 10 comprend un corps 351, un injecteur 302 et une zone d'analyse 354. Une dimension intérieure transversale D354 de la cuve est d'environ 250 microns. Cette dimension peut être un diamètre, si la cuve est de section circulaire, ou un côté, si elle est de section carré ou rectangulaire. Comme illustré par les pointillés, un flux de manchonnage 362 est utilisé pour réduire notablement le diamètre d'un flux échantillon 361, de sorte que dans la zone d'analyse 354 le flux d'échantillon a, dans l'art antérieur, un diamètre D361 inférieur à 50 microns.

La cuve 300, illustrée aux figures 4-6, comprend le corps 301 et l'injecteur 302, disposés sensiblement coaxialement selon un axe d'injection X300. La zone d'analyse 304 est disposée en aval de l'injecteur.

Le corps est réalisé en un matériau injecté, de préférence un matériau plastique. Un tel procédé de fabrication permet d'obtenir des formes complexes. En particulier, une lentille 305 est moulée dans le corps. Cette lentille permet de collecter la lumière occultée, diffractée ou diffusée par les cellules sanguines.

Cette lentille doit avoir des dimensions, notamment un diamètre suffisant pour que les éventuelles inhomogénéités locales dans le matériau injecté soient négligeables relativement à ces dimensions. Dans l'exemple illustré, la lentille 305 à un diamètre voisin de 3mm. Cette lentille injectée est une lentille latérale 305 traversé par le faisceau résultant latéral 315. En outre, la lentille latérale doit permettre de collecter la lumière selon le maximum de directions, c'est-à-dire avec un champ directionnel maximal, Ainsi, plus la lentille est près de l'échantillon, plus le champ directionnel est large. Dans l'exemple illustré, la lentille est une lentille hémisphérique, dite à 90°. De plus, la lentille étant une partie de la paroi de la cuve, il y a directement contact avec le liquide dans la cuve, c'est-à-dire qu'il n'y a pas de lame d'air, à faible indice de réfraction, entre l'échantillon et la lentille. La mesure en est d'autant améliorée.

Pour s'affranchir des défauts d'homogénéité, là où la lumière est particulièrement focalisée on utilise du verre, par exemple un verre de type BK7. C'est notamment le cas pour les fenêtres axiales 306, là où le faisceau source 211 pénètre dans la cuve et là où le faisceau résultant axial 212 en sort.

Pour pouvoir réaliser une lentille injectée d'une telle dimension il convient donc que dans la zone d'analyse, la cuve 300 ait des dimensions au moins comparables. En outre ces grandes dimensions permettent d'intégrer des fenêtres en verre dans des parois en plastique, alors que dans les cuves de l'art antérieur, de petites dimensions, celles-ci sont faites de parois entièrement de verre ou de quartz. Dans l'exemple illustré notamment aux figures 5 et 6, la section intérieure de la cuve est de 4,5mm selon l'axe optique par 3mm selon la direction perpendiculaire. Cette section rectangulaire de grandes dimensions associée à un faible volume de l'échantillon, qui transporte les cellules sanguines à analyser, nécessite l'utilisation d'un manchonnage hydrodynamique de l'échantillon. A titre de comparaison, une cuve de l'art antérieur a Une dimension intérieure transversale D354 de la zone d'analyse voisin de 250 microns.

En amont de la zone d'analyse 304, le corps 301 de la cuve enveloppe l'injecteur 302 et forme autour de l'injecteur le volume de manchonnage 303. Les parois de l'injecteur séparent un flux 311 formé par l'échantillon, à l'intérieur de l'injecteur d'avec un flux de manchonnage 312, dans le volume de manchonnage. Le flux d'échantillon est issu de la branche d'injection 131 du circuit hydraulique 100. Le flux de manchonnage est issu de la branche de manchonnage 135 du circuit hydraulique. Dans la zone d'analyse, les deux flux sont en contact, restent concentriques et s'écoulent simultanément dans la cuve.

Afin de réduire les coûts de fabrication de l'automate, il peut être avantageux de réduire la précision de fabrication des pièces. Comme cela a été dit plus haut, un tel but peut être atteint en créant un flux d'échantillon de plus grande section.

Cependant, si l'on utilise une technique de l'art antérieur où le flux d'échantillon est étiré par un flux de manchonnage, un flux d'échantillon de grande section sera turbulent, ce qui nuirait notablement à la précision des mesures. En outre le flux d'échantillon serait progressivement réduit en section, ce qui va à l'encontre de l'effet recherché, qui est d'avoir un flux d'échantillon de grande section. Un tel but est atteint grâce à la mise en oeuvre du circuit hydraulique 100, précédemment décrit en référence à la figure 1. Un tel circuit permet d'obtenir des vitesses choisies indépendamment pour l'écoulement du flux de manchonnage et pour celui du flux d'échantillon, ceci afin que peu de turbulences apparaissent dans le flux d'échantillon et que ces turbulences n'aient pas d'effet notable sur le résultat de l'analyse. Les deux flux peuvent être chacun sensiblement uniformes, éventuellement laminaires dans certaines gammes de vitesses appropriées.

En outre, un injecteur 302 tel qu'illustré à la figure 7 ou à la figure 8, permet aussi de limiter les turbulences dans le flux d'échantillon. En outre, il permet d'injecter à grande vitesse l'échantillon dans la cuve optique, tout en lui conservant un écoulement sensiblement uniforme.

Un injecteur 302 tel qu'illustré à la figure 7 comprend un tube de structure 320, par exemple en acier inoxydable assurant la rigidité de l'injecteur. Le tube de structure est gainé intérieurement par un tube 321 en un matériau plastique, par exemple un polytétrafluoroéthylène (PTFE). Dans l'exemple illustré les tubes de structure et de gainage sont cylindriques. Le tube de gainage se prolonge, à l'aval du tube de structure, par un bec en le même matériau plastique. Le fait de désolidariser la fonction structurelle du tube de structure, d'avec la fonction d'injection du bec, associé à l'utilisation d'un matériau plastique, permet d'obtenir aisément des formes suffisamment précises à moindre coût.

Le bec a une section progressivement rétrécie depuis un diamètre intérieur D321 du tube de gainage jusqu'à un diamètre intérieur D323 d'un orifice de sortie 323 à une extrémité aval 324 du bec 322. Dans l'exemple décrit, l'extrémité aval 324 est un cylindre de longueur L324. La paroi du bec est d'abord concave vers l'intérieur, puis infléchie pour devenir intérieurement convexe, la section du bec étant ainsi progressivement rétrécie depuis l'amont vers l'aval, depuis le diamètre D321 jusqu'au diamètre D324. La surface concave est tangente avec la surface intérieure du tube de gainage cylindrique. La surface convexe est tangente avec la surface intérieure de l'extrémité cylindrique 324. Dans l'exemple décrit, le diamètre D323 de l'orifice 323 est d'environ 60 microns, le diamètre intérieur D321 du tube de gainage est d'environ 1 millimètre, la longueur L322 du bec est d'environ 2,5 millimètres, celle L320 du tube de structure d'environ 6 millimètres et celle de l'extrémité cylindrique L324 d'environ 200 microns.

Un injecteur 302 tel qu'illustré aux figures 8 et 9 est en une seule pièce et une seule matière sensiblement rigide. Cette matière peut être, par exemple, de l'acier inoxydable, une céramique, un rubis synthétique ou une matière plastique. La matière plastique peut avantageusement être un polytétrafluoroéthylène. L'injecteur comprend un tube sensiblement cylindrique 331 prolongé à l'aval par un bec 332.

Le bec se rétrécit progressivement intérieurement, depuis un diamètre intérieur D331 pour le tube 331, jusqu'à un diamètre intérieur D333 d'un orifice de sortie 333 pour l'échantillon, à une extrémité aval 334 du bec 332. Dans, l'exemple illustré, le rétrécissement se fait selon un tronc de cône ouvert d'un angle compris, de préférence, entre 9 et 10 degrés. Au-delà du tronc de cône est jusqu'à l'orifice de sortie 333, le diamètre reste constant dans une partie cylindrique 335, de longueur L335 et de diamètre D333.

A l'extérieur du bec, le diamètre extérieur en est progressivement moins réduit selon un tronc de côte ouvert d'un angle compris entre 8 et 9 degrés environ, puis, dans l'extrémité nettement plus réduit selon un tronc de côte ouvert d'un angle A334 compris entre 35 et 45 degrés environ, jusqu'à un diamètre extérieur D334 autour de l'orifice de sortie 333. D334 est environ 3 à 4 plus grand que D333.

A titre d'exemple D333=60µm, D334=200µm et A334=40°.

Grâce aux différents dispositifs précédemment décrits, on peut obtenir une vitesse d'injection élevée. Cette dans l'exemple décrit, il est possible d'injecté un échantillon de plus de 200 microlitres en moins de 10 secondes. En particulier, une telle vitesse d'injection permet d'utiliser un taux de dilution important de l'échantillon de sang, sans augmenter la durée de l'analyse relativement à des automates de l'art antérieur. En particulier, une même dilution, par exemple 1/160ème, peut être utilisé pour l'analyse de l'hémoglobine par le dispositif 110 (voir figure 3) et pour l'analyse des globules blancs par le dispositif optique 120, au lieu de 1/80ème généralement utilisée pour l'analyse des globules blancs.

Les figures 11a-c illustrent les résultats obtenus en mettant en oeuvre le procédé selon l'invention, et l'appareillage ledit appareillage utilisant une cuve optique 7 et un dispositif optique 8. La figure 11a montre un test de linéarité positif de la mesure de l'hémoglobine et met donc en évidence la mesure possible et fiable du taux d'hémoglobine d'un échantillon de sang selon l'invention. La figure 11b montre une matrice optique obtenue sur un échantillon test de sang à 30% d'éosinophiles dans lequel on a ajouté la formulation selon le procédé de l'invention. Sur cette matrice, les cinq sous populations sont effectivement présentes et différenciées (ensembles délimités sur le cytographe : E pour éosinophiles, N pour neutrophiles, M pour monocytes, B pour basophiles et L pour lymphocytes). La figure 11c montre le test de linéarité positif sur la mesure par résistivité du taux de globules blancs.

Ces figures montrent que grâce à l'invention, on peut réaliser une analyse au moins du taux d'hémoglobine, du taux de globules blancs et une différentiation leucocytaire en utilisant la formulation défini dans les revendisations, notamment sous forme de mono-réactif.

Bien sûr, l'invention n'est pas limitée aux exemples qui viennent d'être décrits et de nombreux aménagements peuvent être apportés à ces exemples sans sortir du cadre de l'invention.

Par exemple, d'autres produits que le diluant ou le réactif peuvent être utilisés pour respectivement former le flux de manchonnage et le piston fluide, particulièrement s'ils sont disponibles dans l'automate notamment pour d'autres utilisations.

Aussi, au lieu d'être uniquement disposée sur le circuit d'injection, une résistance fluidique peut être disposée sur le circuit de manchonnage ou sur les deux à la fois. Ceci peut être fait en fonction du débit maximum donné par les moyens de déplacement des liquides destinés respectivement au pistonnage ou au manchonnage.

Plusieurs ou toutes les lentilles de la cuve optique et/ou du dispositif optique peuvent aussi être réalisées injectées avec le corps de la cuve, au lieu d'une seule tel que précédemment illustré. En particulier, les fenêtres en verre peuvent être injectées. Particulièrement si les inhomogénéités dans le matériau injecté sont sensiblement négligeables en regard de la précision voulue pour les mesures.

Un dispositif de réglage et/ou les moyens séparateurs précédemment décrits peuvent être utilisés indépendamment les uns des autres et éventuellement avec une autre source lumineuse qu'une diode électroluminescente.

## Revendications

1. Procédé d'analyse automatique d'un échantillon de sang, dans lequel :
* on forme, dans un bac unique dit de dilution et d'analyse, une solution d'analyse contenant :
- ledit échantillon de sang,
- un diluant,
- un mono réactif comprenant au moins un composé pour lyser les globules rouges, au moins un composé pour stabiliser l'hémoglobine sous forme d'oxyhémoglobine et au moins un composé pour protéger les globules blancs, permettant la discrimination d'au moins cinq principales sous-populations leucocytaires, ledit composé pour protéger les globules blancs étant choisi parmi les bétaïnes et sulfo-bétaïnes d'ammoniums quaternaires, les oxydes d'aminés tertiaires, les composés de type glycosidiques et les composés de type glucidiques,
* on mesure sur cette solution d'analyse dans ledit bac après la lyse des globules rouges le taux d'hémoglobine par spectrophotométrie ; et
* on prélève une quantité appropriée de cette solution d'analyse dans ledit bac sur laquelle on réalise une différenciation leucocytaire d'au moins 5 sous-populations par un moyen optique.

2. Procédé selon la revendication 1 **caractérisé en ce** l'on ajoute en outre au mono-réactif un colorant ou un mélange de colorants pour marquer spécifiquement au moins une sous-population de leucocytes.

3. Procédé selon l'une des revendications 1 à 2, **caractérisé en ce** le mono-réactif est adapté à réaliser la fonction de diluant pour réaliser une mono-dilution de l'échantillon de sang.

4. Procédé selon l'une des revendications 1 à 3, **caractérisé en ce que** la solution d'analyse présente un taux de dilution de l'échantillon de sang adapté à la mesure de l'hémoglobine par spectrophotométrie.

5. Procédé selon la revendication 4, **caractérisé en ce que** le taux de dilution de la solution d'analyse est compris entre 1/100^{e} et 1/500^{e}, de préférence entre 1/160^{e} et 1/180^{e}_{.}

6. Procédé selon la revendication 1, **caractérisé en ce que** le prélèvement d'une quantité appropriée de solution d'analyse pour réaliser la différentiation leucocytaire est effectué préalablement à la mesure de l'hémoglobine dans ledit bac, le taux de dilution de la solution de mesure prélevée étant adapté à ladite mesure par ledit moyen optique, et **en ce que** l'on ajoute à ladite solution d'analyse restante du diluant de manière à augmenter la dilution dans ledit bac pour obtenir une dilution adaptée à une mesure de l'hémoglobine par spectrophotométrie.

7. Procédé selon la revendication 1 **caractérisé en ce que** le moyen optique étant un cytomètre de flux de type hydrofocus, le taux de dilution de la solution d'analyse prélevée pour la mesure par cytomètre est inférieur à 1/100^{e}.

8. Procédé selon la revendication 1, **caractérisé en ce que** le moyen optique est un cytomètre à manchonnage passif, le taux de dilution de la solution d'analyse prélevée pour la mesure par cytomètre étant supérieur à 1/100^{e}.

9. Procédé selon la revendication 7 ou 8, **caractérisé en ce que** le taux de dilution de la solution d'analyse pour la mesure de l'hémoglobine par spectrophotométrie est compris entre 1/100^{e} et 1/500^{e}.

10. Procédé selon l'une quelconque des revendications précédentes, **caractérisé en ce que** dans une étape préalable à l'ajout desdits composés, on prélève une fraction d'une pré-solution constituée de l'échantillon de sang et du diluant sur laquelle on réalise un comptage des globules rouges et/ou des plaquettes par résistivité.

11. Procédé selon l'une quelconque des revendications précédentes, **caractérisé en ce que** le composé de lyse est un surfactant ionique choisi parmi :
- les sels d'ammoniums quaternaires, de préférence les sels d'alkyltriméthylammoniums et plus particulièrement encore les bromures et chlorures de cétyl-, dodécyl-, tétradécyl- et hexadécyltriméthylammonium ;
- les sels de pyridiniums ;
- les aminés à longues chaînes éthoxylées ; et
- les alkylsulfates (SDS).

12. Procédé selon l'une quelconque des revendications précédentes, **caractérisé en ce que** le composé stabilisant l'hémoglobine sous la forme d'un complexe oxyhémoglobine est de préférence choisi parmi :
- les chélates mono ou polydendates présentant des atomes ligands (doublets non liants : O, N, S, et groupement carboxy COO-...),
- les composés aromatiques (chélates mono ou polydentates) comportant des atomes ligands (présentant des doublets non liant : O, N, S...), et
- les saponines, les oxydes d'aminés tertiaires, les bétaïnes et sulfo-bétaïnes d'ammoniums quaternaires.

13. Procédé selon l'une quelconque des revendications précédentes, **caractérisé en ce que** l'on ajoute en outre à la solution d'analyse un fixateur de la membrane des globules blancs.

14. Procédé selon l'une quelconque des revendications précédentes, **caractérisé en ce que** l'on ajoute en outre à la solution d'analyse un tampon pH capable de fixer le pH de la solution d'analyse entre 5,0 et 8,0, de préférence un pH de 7.

15. Procédé selon l'une quelconque des revendications précédentes, **caractérisé en ce que** l'on ajoute en outre à la solution d'analyse un sel de fond pour assurer sa conductivité.

## Claims

1. Method for the automatic analysis of a blood sample in which:
* an analysis solution is formed in a single dilution and analysis tank, the analysis solution containing:
- said blood sample,
- a diluent,
- a mono-reagent comprising at least one compound to lyse the erythrocytes, at least one compound to stabilize the haemoglobin in the form of oxyhaemoglobin and at least one compound to protect the leucocytes, allowing the distinguishing of at least five main leucocyte sub-populations, said compound to protect the leucocytes being chosen from betaines and sulphobetaines of quaternary ammoniums, tertiary amine oxides, glycosidic type compounds and.glucidic type compounds,
* the haemoglobin level is measured in this analysis solution by spectrophotometry in said tank after the lysis of the erythrocytes; and
* an appropriate quantity of this analysis solution is taken from said tank on which a leucocytic differentiation of at least five sub-populations is carried out by an optical means.

2. Method according to claim 1, **characterized in that** a dye or mixture of dyes specifically labelling at least one leucocyte sub-population is also added to the mono-reagent.

3. Method according to one of claims 1 to 2, **characterized in that** the mono-reagent is suitable to carry out the function of diluent in order to produce a mono-dilution of the blood sample.

4. Method according to one of claims 1 to 3, **characterized in that** the analysis solution has a dilution rate of the blood sample suitable for measuring the haemoglobin by spectrophotometry.

5. Method according to claim 4, **characterized in that** the dilution rate of the analysis solution is comprised between 1/100^{th} and 1/500^{th}, and preferably between 1/160^{th} and 1/180^{th}.

6. Method according to claim 1, **characterized in that** the sampling of an appropriate quantity of analysis solution to carry out the leucocyte differentiation is carried out before the measurement of the haemoglobin in said tank, the dilution rate of the sampled measuring solution being suitable for said measurement by said optical means, and **in that** diluent is added to said remaining analysis solution so as to increase the dilution in said tank in order to obtain a dilution suitable for a measurement of the haemoglobin by spectrophotometry.

7. Method according to claim 1, **characterized in that** the optical means is a hydrofocus-type flow cytometer, and the dilution rate of the sampled analysis solution for measurement by cytometry is less than 1/100^{th}.

8. Method according to claim 1, **characterized in that** the optical means is a cytometer with passive sleeving, and the dilution rate of the sampled solution for measurement by cytometry is greater than 1/100^{th}.

9. Method according to claim 7 or 8, **characterized in that** the dilution rate of the measuring solution for the measurement of the haemoglobin by spectrophotometry is comprised between 1/100^{th} and 1/500^{th}.

10. Method according to any one of the previous claims, **characterized in that** in a stage before the addition of said compounds, a fraction of a pre-solution is taken constituted by the sample of blood and diluent on which counting of the erythrocytes and/or platelets is carried out by resistivity.

11. Method according to any one of the previous claims, **characterized in that** lysis compound is an ionic surfactant chosen from:
- the quaternary ammonium salts, preferably alkyltrimethylammonium salts and still more particularly cetyl-, dodecyl-, tetradecyl- and hexadecyltrimethylammonium bromides and chlorides;
- pyridinium salts;
- long-chain ethoxylated amines; and
- alkyl sulphates (SDS).

12. Method according to any one of the previous claims, **characterized in that** the compound which stabilizes the haemoglobin in the form of a oxyhaemoglobin complex is preferably chosen from:
- mono or polydentate chelates presenting ligand atoms (non-binding pairs: O, N, S, and carboxy COO- groups etc.),
- the aromatic compounds (mono or polydentate chelates) comprising ligand atoms (having non-binding pairs: O, N, S etc.), and
- saponins, tertiary amine oxides, betaines and sulphobetaines of quaternary ammoniums.

13. Method according to any one of the previous claims, **characterized in that** a fixing agent of the membrane of the leucocytes is also added to the analysis solution.

14. Method according to any one of the previous claims, **characterized in that** a pH buffer capable of setting the pH of the analysis solution between 5.0 and 8.0, preferably at a pH of 7, is also added to the analysis solution.

15. Method according to any one of the previous claims, **characterized in that** a background salt is also added to the analysis solution to ensure its conductivity.

## Patentansprüche

1. Verfahren zur automatischen Analyse einer Blutprobe, bei dem:
* man in einem einzigen Behälter, Verdünnungs- und Analysebehälter genannt, eine Analyselösung bildet, die enthält:
- die Blutprobe,
- ein Verdünnungsmittel,
- einen monoreaktiven Stoff, der mindestens umfasst eine Verbindung zum Lysieren der roten Blutkörperchen, mindestens eine Verbindung zum Stabilisieren des Hämoglobins in Form von Oxyhämoglobin und mindestens eine Verbindung zum Schützen der weißen Blutkörperchen und der die Diskriminierung von mindestens fünf Leukozyten-Unterpopulationen gestattet, wobei diese Verbindung zum Schützen der weißen Blutkörperchen ausgewählt ist aus den quaternären Ammoniumbetainen und -sulfobetainen, den tertiären Aminoxiden, den Verbindungen vom Typ Glykosidverbindungen und den Verbindungen vom Typ Kohlenhydratverbindungen,
* man an dieser Analyselösung in dem Behälter nach der Lyse der roten Blutkörperchen den Hämoglobingehalt durch Spektrophotometrie misst; und
* man eine geeignete Menge dieser Analyselösung in dem Behälter entnimmt, an der man eine Leukozytendifferenzierung von mindestens 5 Unterpopulationen mit einem optischen Mittel vornimmt.

2. Verfahren nach Anspruch 1, **dadurch gekennzeichnet, dass** man dem monoreaktiven Stoff einen Farbstoff oder eine Mischung von Farbstoffen zusetzt, um mindestens eine Leukozyten-Unterpopulation spezifisch zu markieren.

3. Verfahren nach einem der Ansprüche 1 bis 2, **dadurch gekennzeichnet, dass** der monoreaktive Stoff dafür ausgelegt ist, die Verdünnungsmittelfunktion zur Herstellung einer Monoverdünnung der Blutprobe zu realisieren.

4. Verfahren nach einem der Ansprüche 1 bis 3, **dadurch gekennzeichnet, dass** die Analyselösung einen Verdünnungsgrad der Blutprobe aufweist, der an die Messung des Hämoglobins durch Spektrophotometrie angepasst ist.

5. Verfahren nach Anspruch 4, **dadurch gekennzeichnet, dass** der Verdünnungsgrad der Analyselösung zwischen einem Hundertstel und einem Fünfhundertstel, vorzugsweise zwischen einem Einhundertsechzigstel und einem Hundertachtzigstel liegt.

6. Verfahren nach Anspruch 1, **dadurch gekennzeichnet, dass** die Entnahme einer geeigneten Menge an Analyselösung zur Durchführung der Leukozytendifferenzierung vor der Messung des Hämoglobins in dem Behälter vorgenommen wird, wobei der Verdünnungsgrad der entnommenen Messlösung an die Messung durch das optische Mittel angepasst ist, und dass man der verbleibenden Analyselösung Lösungsmittel so zusetzt, dass die Verdünnung in dem Behälter erhöht wird, um eine an eine Messung des Hämoglobins durch Spektrophotometrie angepasste Verdünnung zu erhalten.

7. Verfahren nach Anspruch 1, **dadurch gekennzeichnet, dass** das optische Mittel ein Flusscytometer vom Typ Hydrofocus ist, wobei der Verdünnungsgrad der für die Messung mit dem Cytometer entnommenen Analyselösung niedriger als ein Hundertsel ist.

8. Verfahren nach Anspruch 1, **dadurch gekennzeichnet, dass** das optische Mittel ein Cytometer mit passiver Umhüllung ist, wobei der Verdünnungsgrad der für die Messung mit dem Cytometer entnommenen Analyselösung höher als ein Hundertstel ist.

9. Verfahren nach Anspruch 7 oder 8, **dadurch gekennzeichnet, dass** der Verdünnungsgrad der Analyselösung für die Messung des Hämoglobins durch Spektrophotometrie zwischen einem Hundertstel und einem Fünfhundertstel liegt.

10. Verfahren nach einem der vorhergehenden Ansprüche, **dadurch gekennzeichnet, dass** man in einem der Zugabe der Verbindungen vorausgehenden Schritt eine Fraktion einer aus der Blutprobe und aus dem Verdünnungsmittel bestehenden Vorlösung entnimmt, an der man eine Zählung der roten Blutkörperchen und/oder der Blutplättchen durch Resistivität vornimmt.

11. Verfahren nach einem der vorhergehenden Ansprüche, **dadurch gekennzeichnet, dass** die Lyseverbindung ein Ionentensid ist, das ausgewählt ist aus:
- den quaternären Ammoniumsalzen, vorzugsweise den Alkyltrimethylammoniumsalzen und insbesondere den Ketyl-, Dodecyl-, Tetradecyl- und Hexadecyltrimethylammoniumbromiden und -chloriden;
- den Pyridiniumsalzen;
- den Aminen mit langen ethoxylierten Ketten; und
- den Alkylsulfaten /SDS).

12. Verfahren nach einem der vorhergehenden Ansprüche, **dadurch gekennzeichnet, dass** die das Hämoglobin in Form eines Oxyhämoglobinkomplexes stabilisierende Verbindung vorzugsweise ausgewählt ist aus:
- den mono- oder polydendaten Chelaten mit Ligandatomen (nichtbindende Dubletts: O, N, S und Carboxygruppe COO-...),
- den aromatischen Verbindungen (mono- oder polydendate Chelate) mit Ligandatomen (mit nichtbindenden Dubletts: O, N, S...), und
- den Saponinen, den tertiären Aminoxiden, den quaternären Ammoniumbetainen und -sulfobetainen.

13. Verfahren nach einem der vorhergehenden Ansprüche, **dadurch gekennzeichnet, dass** man der Analyselösung außerdem ein Mittel zur Fixierung der Membrane der weißen Blutkörperchen zusetzt.

14. Verfahren nach einem der vorhergehenden Ansprüche, **dadurch gekennzeichnet, dass** man der Analyselösung außerdem einen pH-Puffer zusetzt, der den pH-Wert der Analyselösung zwischen 5,0 und 8,0, vorzugsweise einen pH-Wert von 7, fixieren kann.

15. Verfahren nach einem der vorhergehenden Ansprüche, **dadurch gekennzeichnet, dass** man der Analyselösung außerdem ein Grundsalz zusetzt, um ihre Leitfähigkeit zu gewährleisten.
